(19) Europäisches Patentamt
European Patent Office
Office européen des brevets

(11) EP 1 516 878 A1

(12) **EUROPEAN PATENT APPLICATION**
published in accordance with Art. 158(3) EPC

(43) Date of publication:
**23.03.2005 Bulletin 2005/12**

(21) Application number: **03738516.8**

(22) Date of filing: **26.06.2003**

(51) Int Cl.[7]: **C07D 307/84**, A61K 31/343, A23L 1/30, A23L 1/28, A23L 2/00, C12C 5/00, C12G 3/00, A61K 35/72, A61K 35/78, A61P 1/00, A61P 1/14, A61P 13/02, A61P 43/00

(86) International application number:
**PCT/JP2003/008081**

(87) International publication number:
**WO 2004/002978 (08.01.2004 Gazette 2004/02)**

(84) Designated Contracting States:
**AT BE BG CH CY CZ DE DK EE ES FI FR GB GR HU IE IT LI LU MC NL PT RO SE SI SK TR**

(30) Priority: **26.06.2002 JP 2002186029**
**17.01.2003 JP 2003010426**

(71) Applicant: **SUNTORY LIMITED**
**Osaka-shi, Osaka 530-8203 (JP)**

(72) Inventors:
- **SUWA, Yoshihide**
**Ibaraki-shi, Osaka 567-0009 (JP)**
- **FUJII, Wataru**
**Ibaraki-shi, Osaka 567-0031 (JP)**
- **HORI, Hisako**
**Suita-shi, Osaka 565-0817 (JP)**
- **YOKOO, Yoshiaki**
**Ibaraki-shi, Osaka 567-0815 (JP)**
- **NUKAYA, Haruo**
**Shizuoka-shi, Shizuoka 424-0884 (JP)**
- **TSUJI, Kuniro**
**Shizuoka-shi, Shizuoka 422-8005 (JP)**

(74) Representative: **Albrecht, Thomas, Dr.**
**Kraus & Weisert,**
**Thomas-Wimmer-Ring 15**
**80539 München (DE)**

# (54) REFRESHMENT CAPABLE OF STIMULATING MOVEMENT OF DIGESTIVE TRACT

(57) A substance capable of stimulating the movement of digestive tract, comprising a compound of the formula: (I) (active substance α and active substance β being stereoisomers), which substance is derived from natural products and hence cause no side effects, is highly safe even when continuously used for a prolonged period of time and can be widely employed as additives to refreshments, etc.; and a refreshment containing an additive comprising the stimulating substance. In particular, a digestive tract movement stimulating substance capable of acting on muscarine $M_3$ receptor, obtained from beer through isolation and purification, which substance is one having drinkability stimulating effect; and a composition from natural products or natural processed products, comprising the above substance. The thus provided substance or composition is used as an additive to refreshment, thereby providing a refreshment capable of stimulating the movement of digestive tract.

Fig. 15

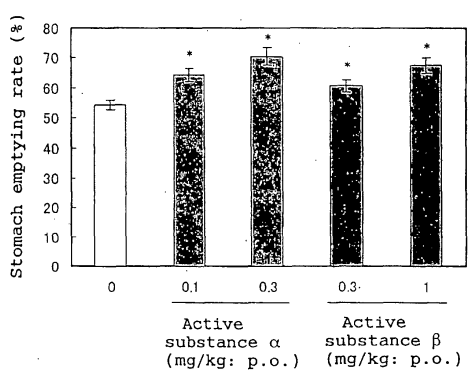


EP 1 516 878 A1

## Description

TECHNICAL FIELD

The present invention relates to substances that act on muscarinic $M_3$ receptors and, more particularly, to substances that have an ability to stimulate gastrointestinal motility, stomach acid secretion, urination or appetite, or an ability to enhance drinkability.

The present invention also relates to food and beverage products that make use of such substances, or natural products, processed natural products or compositions containing such substances and, in particular, to food and beverage products that take advantage of the ability of the substances to stimulate gastrointestinal motility or the ability to enhance drinkability.

TECHNICAL BACKGROUND

Foodstuffs ingested by a human are subjected to various digestive processes in different regions of the gastrointestinal system and most nutrients are absorbed in the small intestine before the ingested materials are excreted. In order for these digestion and absorption processes to take place in an orderly manner, the foodstuffs need to be successively propelled through the gastrointestinal tract depending on their stage of digestion. Thus, the gastrointestinal motility is an important physiological function that governs various digestive processes of the gastrointestinal tract, including transportation, storage, stirring/mixing, and excretion of the contents.

The contents of the gastrointestinal tract are transported, stored, and stirred/mixed by the gastrointestinal motility, which is a coordinated contraction (peristalsis) of smooth muscles forming the wall of the gastrointestinal tract. The gastrointestinal motility is under nervous regulation and humoral regulation. The nervous regulation consists of the dual control by the autonomic nervous system and the control by the intramural nerves of the gastrointestinal walls. The principal nerve that takes part in the nervous regulation is the cholinergic parasympathetic postganglionic fiber. In comparison, the humoral regulation is effected by the gastrointestinal hormones.

If the balance among these regulation mechanisms is disrupted and the gastrointestinal motility is interfered, then each stage of the digestive process is interrupted, leading to difficulty in swallowing, pyrosis, nausea, vomiting, abdominal pain, abdominal swelling, diarrhea, constipation, and other gastrointestinal disorders. In particular, the number of patients who suffer from these symptoms but do not have any organic diseases such as ulcers and cancers is on the rise in our modern stressful society. In Japan, such patients have been diagnosed as having chronic gastritis and have been given no practical treatments. However, the advancement in the diagnosis of gastrointestinal diseases has revealed that many of these cases are caused by defective gastrointestinal motility, as represented by delayed emptying of stomach, and the terms "functional dyspepsia" and "non-ulcer dyspepsia (NUD)" have been coined to refer to these disorders and distinguish them from gastritis. Such diseases also include gastric atonia, a disorder common in older people caused by a decreased tone of muscles in stomach wall, and other disorders caused by psychophysiological factors, such as nervous dyspepsia and stomach neuropathy. The postoperative reduction in the gastrointestinal motility and loss of appetite caused by chemotherapy are also becoming a social concern.

The recent progress in the study of receptors involved in the gastrointestinal motility has unveiled detailed mechanisms of the nervous regulation of the gastrointestinal motility. The gastrointestinal motility is largely regulated by intramural cholinergic nerves, which are parasympathetic postganglionic fibers. Acetylcholine released from the synaptic terminals of these nerves bind to muscarinic $M_3$ receptors of smooth muscles to bring about contraction of the muscles. As acetylcholine is degraded by cholinesterase, the smooth muscles return to their original length. An increase in the frequency of this contraction and relaxation facilitates the gastrointestinal motility.

Thus, a substance that can act as an agonist of muscarinic $M_3$ receptors can cause contraction of smooth muscles in the gastrointestinal tract and thereby stimulate the gastrointestinal motility. Such a substance can be used to ameliorate symptoms of the gastrointestinal disorders caused by reduced gastrointestinal motility and can thus serve as a highly effective stimulant of gastrointestinal motility.

Alcoholic beverages, among other things, are known to affect the motility of upper gastrointestinal tract. For example, it has been demonstrated that beer promotes the emptying of stomach and reduces the time it takes for the stomach contents to pass through to the appendix, whereas an ethanol solution having a similar alcohol concentration to beer (7.5%) inhibits stomach emptying (Pfeiffer *et al., Clin. Invenstig,.* vol. 70, pp. 487-491, 1992). An interesting finding has been reported that a particular type of beer that is cleared from the stomach more quickly than the other types of beer tends to show a higher drinkability (*Biosci. Biotechnol. Biochem,* Vol.62, pp.846-851, 1998).

As used herein, the term "drinkability of beer" refers to the beer's ability to remain refreshing after consumed in substantial amounts. In Europe, the term has been known as "weitertrinken" or "drinkability" and is considered to be an important factor in evaluating beers. The results of the above-described studies suggest that beer contains, aside from its alcohol component, certain substances that can stimulate the gastrointestinal motility and promote its

drinkability. Such active ingredients have never been thoroughly studied, nor have they been identified.

Using mice, the present inventors have further demonstrated that the dried product of beer has an ability to promote stomach emptying. The present inventors have also examined the mechanism of action of the dried beer product by conducting the receptor binding tests and the contractility tests using longitudinal muscles of mouse ilea. As a result, the dried beer products have proven to have an ability to stimulate muscarinic $M_3$ receptors and thereby stimulate gastrointestinal motility(*Alcoholism: Clinical & Experimental Research,* Vol.26, No.5, pp 677-681, 2002).

If a stimulant of gastrointestinal motility or an enhancer of drinkability that acts on muscarinic $M_3$ receptors is identified in beer, such a stimulant or enhancer can be used to enhance drinkability and stimulate the gastrointestinal motility, gastric acid secretion, urination or appetite. Thus, such stimulants or enhancers are expected to be highly useful.

Various studies have been conducted to examine trace components existing in the materials of beer, such as barley: US patent No. 3,475,459 describes hordatine A, a fungicide obtained from barley. Its three-dimensional structure, however, has never been closely examined in this patent based on the relevant data, nor has any stereoisomers been identified. Further, nothing is mentioned about the activities of hordatine A besides its antifungal activity, in particular, activity toward muscarinic $M_3$ receptors.

Accordingly, it is an objective of the present invention to isolate and identify active ingredients in beer that can act on muscarinic $M_3$ receptors and stimulate gastrointestinal motility. It is another objective of the present invention to apply such substances to a wide range of functional food and beverage products to thereby provide enhancers of drinkability, and stimulants of gastrointestinal motility, gastric acid secretion, urination, or appetite.

During the course of our studies, the present inventors have attempted to purify a stimulant of gastrointestinal motility from beer based on its binding activity to muscarinic $M_3$ receptors and have eventually succeeded in isolating the substance in a four-step liquid chromatography. The present inventors have also determined the structure of the substance and thus have completed the present invention.

DISCLOSURE OF THE INVENTION

In one aspect, the present invention concerns a compound represented by the following chemical formula (I):

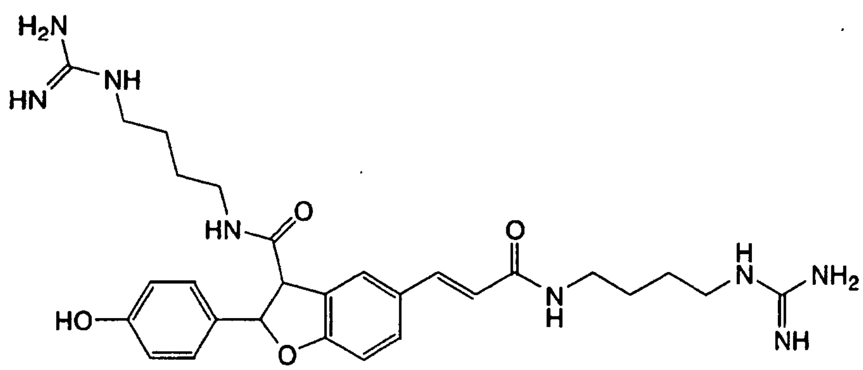

(I)

In this aspect of the invention, the three-dimensional structure of the compound is determined and the compound is actually provided.

Specifically, the present inventors determined the three dimensional structure of the compound of the chemical formula (I) by first separating two isomers, which we refer to as active substances α and β, and then subjecting each of the active substances α and β to analysis by UV-absorption spectroscopy, mass spectrometry, nuclear magnetic resonance spectroscopy, and other analytical techniques.

Specifically, the active substances α and β, each an isomer of the compound of the chemical formula (I), are represented by the following chemical formulas (II) and (III), respectively:

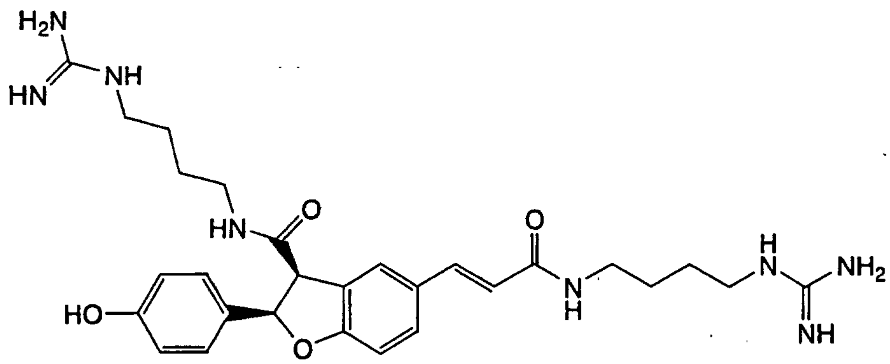

(II)

and

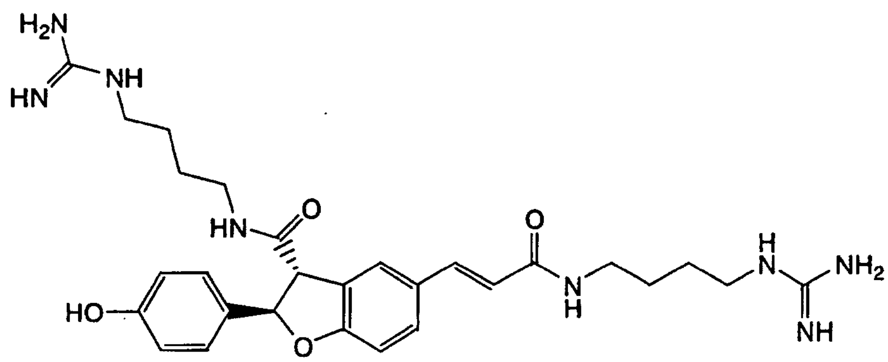

(III)

The compound of the chemical formula (I) according to the present invention contains two chiral carbon atoms in its molecule. Therefore, the compound of the chemical formula (I) has different stereoisomers, which are each encompassed by the chemical formula (I). What is represented by the chemical formula (I) also encompasses mixtures of the stereoisomers of the compound of the chemical formula (I).

The compound represented by the chemical formula (II) (*cis*-form) and the compound represented by the chemical formula (III) (*trans*-form), each a stereoisomer of the compound of the chemical formula (I), have respective enantiomers. The active substances $\alpha$ and $\beta$ of the present invention have absolute configurations that give CD spectra shown in Figs. 13 and 14, respectively. These enantiomers are encompassed by the compounds represented by the chemical formulae (II) and (III).

The compounds represented by the chemical formulae (I) through (III) of the present invention may take the form of salt. Throughout the description and claims of the present invention, such salts are also encompassed by the compounds represented by the chemical formulae (I) through (III).

The compounds represented by the chemical formulae (I) through (III) are dimers of p-coumaroylagmatine. p-coumaroylagmatine contains a double bond between its phenol group and amide group. If both of the two p-coumaroylagmatine molecules that form a dimer are in cis-form, then the resulting dimer has a cis configuration with respect to the five-membered ring as shown in the chemical formula (III).

In comparison, the compound of the chemical formula (II) has a cis configuration with respect to the five-membered ring and a trans configuration with respect to the double bond. This is a rare structure that can hardly be expected from dimerization of two cis molecules or dimerization of two trans molecules. In other words, the present inventors have discovered that the compound of the chemical formula (II) has an extremely rare structure for naturally occurring compounds.

In another aspect, the present invention provides a method for separating/purifying the active substances $\alpha$ and $\beta$. Specifically, the method includes freeze-drying beer, which serves as the starting material, to remove volatile components and collect non-volatile components; subjecting the non-volatile components to a chromatography using synthetic absorbent to remove unadsorbed components and collect adsorbed components in a crude fraction containing concentrated active substances; subjecting the crude fraction to a weakly acidic cation exchange chromatography to remove unadsorved components; eluting the active substances with acidic methanol (methanol/hydrochloric acid); subjecting the eluate to gel filtration chromatography using 0.01N hydrochloric acid to obtain a purified fraction; subjecting the purified fraction to $C_{18}$ high performance liquid chromatography (ODS-HPLC) for further purification to isolate the two active substances. The active substances so obtained were water-soluble.

In HPLC, each of the two active substances α and β was determined to give a single peak. Each was determined to have an affinity for muscarinic $M_3$ receptors in the muscarinic $M_3$ receptor binding test. Specifically, the active substances α and β were tested in mice for their ability to promote stomach emptying to give a measure of their effects on gastrointestinal motility. As a result, each of them proved to have a strong ability to promote gastrointestinal motility.

Muscarinic $M_3$ receptors are present not only in small intestines but also on the surface of various cells of stomach and play a significant role in eliciting physiological activities of stomach. Specifically, mural cells, chief cells, and G cells are stimulated by acetylcholine via muscarinic $M_3$ receptors to secrete gastric acid, pepsinogen, and gastrin, respectively. This brings about the contraction of smooth muscles of stomach.

Muscarinic $M_3$ receptors are also present in smooth muscles (detrusor muscles) of bladder and when acted by acetylcholine, cause contraction of the smooth muscles, facilitating urination.

Capable of acting on muscarinic $M_3$ receptors, the active substances α and β of the present invention can stimulate, in addition to gastrointestinal motility, urination, gastric acid secretion, and appetite, for which muscarinic $M_3$ receptors are responsible. For this reason, these substances may find applications in antiemetics or snacks for those who suffer taste repellency.

In another aspect, the present invention provides the active substances represented by the chemical formulae (I) to (III) that can enhance drinkability and can promote gastrointestinal motility, gastric acid secretion, urination, and appetite.

In an effort to find the source of the active substances α and β, the present inventors have screened various materials for the presence of these substances and have found that the active substances are abundant in fermentation products of yeast, barley, germinated barley, and fractionated products of germinated barley. The present inventors have also found that the active substances are particularly abundant in barley malt sprouts, a fractionated product of germinated barley.

Based on this finding, the present inventors examined, by using Magnus' method, extracts of barley malt sprouts, rich sources of the active substances, for their effects on the contraction of longitudinal muscles of guinea pig ilea. The tests indicated that the extracts elicited strong contraction of the longitudinal muscles of ilea, thereby stimulating gastrointestinal motility.

In still another aspect, the present invention provides various food and beverage products, additives for use in food and beverage products, pharmaceutical products, and animal feeds that make use of the active substances represented by the chemical formulae (I) through (III), or naturally-occurring materials, processed products of naturally-occurring materials, or compositions that contain the active substances. Such products can stimulate gastrointestinal motility and enhance drinkability.

Preferably, the food and beverage products, additives for use in food and beverage products, pharmaceutical products, and animal feeds of the present invention contain barley malt sprouts.

More preferably, the food and beverage products of the present invention are provided in the form of alcoholic beverages, non-alcoholic beverages, health food products, and specially designed food products.

## BRIEF DESCRIPTION OF THE DRAWINGS

Fig. 1 is a graph showing an inhibition curve obtained in Reference Example 1, which indicates inhibition of the binding of [$^3$H]4-DAMP by a fractionated product obtained from dried beer products.

Fig. 2 is a flowchart showing a separation/purification process of Example 1, by which to separate/purify, from beer, a substance capable of stimulating gastrointestinal motility.

Fig. 3 is an HPLC chromatogram of an active substance α of the present invention obtained in Example 1.

Fig. 4 is a graph showing an inhibition curve obtained in Example 1, which indicates inhibition of the binding of [$^3$H]4-DAMP by the active substance α of the present invention.

Fig. 5 is a graph showing an inhibition curve obtained in Example 1, which indicates inhibition of the binding of [$^3$H]4-DAMP by an active substance β of the present invention.

Fig. 6 shows the results of UV-spectrophotometry indicating UV-absorbance of the active substances α and β of Example 2.

Fig. 7 shows the results of mass spectrometry using the active substance α of Example 2.

Fig. 8 shows the results of mass spectrometry using the active substance β of Example 2.

Fig. 9 shows the results of nuclear magnetic resonance ($^1$H-NMR) spectrometry using the active substance α of Example 2.

Fig. 10 shows the results of nuclear magnetic resonance ($^1$H-NMR) spectrometry using the active substance β of

Example 2.

Fig. 11 shows the results of nuclear magnetic resonance ($^{13}C$-NMR) spectrometry using the active substance $\alpha$ of Example 2.

Fig. 12 shows the results of nuclear magnetic resonance ($^{13}C$-NMR) spectrometry using the active substance $\beta$ of Example 2.

Fig. 13 shows the results of CD spectrometry using the active substance $\alpha$ of Example 2.

Fig. 14 shows the results of CD spectrometry using the active substance $\beta$ of Example 2.

Fig. 15 is a graph showing the results of the experiment conducted in Example 3 in which the active substances $\alpha$ and $\beta$ were examined for their ability to promote stomach emptying in mice.

Fig. 16 is an HPLC chromatogram of a composition extracted and partially purified from barley malt sprouts in Example 4.

Fig. 17 is a graph showing the results of Example 5 in which a barley malt sprout extract containing the active substance was examined for its ability to bring about contraction of longitudinal muscles of guinea pig ileum.

Fig. 18 is a graph showing the results of Example 6 in which a food and beverage product (alcoholic beverage) containing a substance capable of stimulating gastrointestinal motility was examined in humans for its ability to stimulate urination.

Fig. 19 is a graph showing the results of Example 7 in which a food and beverage product (non-alcoholic beverage) containing the substance capable of stimulating gastrointestinal motility was examined in rats for its ability to enhance drinkability.

Fig. 20 is a graph showing the results of Example 8 in which a food and beverage product (beer) containing the substance capable of stimulating gastrointestinal motility was examined in humans for its ability to enhance drinkability.

BEST MODE FOR CARRYING OUT THE INVENTION

The present invention will now be described in detail with respect to isolation, purification, and properties of active substances of the present invention, which are capable of acting on muscarinic $M_3$ receptors and are represented by the chemical formulae (I) through (III).

Specifically, the active substances of the present invention, which are capable of acting on muscarinic $M_3$ receptors and are represented by the chemical formulae (I) through (III), can be isolated and purified from beer by using a four-step liquid chromatography in proper combination. More specifically, beer is freeze-dried to remove volatile components, and the remaining non-volatile components are subjected to a chromatography using such synthetic adsorbents as AMBERLITE XAD-2 and DIAION HP-20 to remove unadsorbed components. The adsorbed components are then eluted with an alcohol, such as methanol, to obtain a crude fraction containing concentrated active substances.

Subsequently, the resulting alcohol eluate is subjected to a weakly acidic cation exchange chromatography, for example, CM-sephadex C-25 column chromatography, to remove unadsorbed components, and the adsorbed active substances are eluted with an acidic methanol (methanol/hydrochloric acid).

The resulting eluate is then subjected to a gel filtration chromatography using 0.01N hydrochloric acid to obtain a purified fraction, which in turn is subjected to a $C_{18}$ high performance liquid chromatography (ODS-HPLC) for further purification to isolate the desired two active substances (the active substance $\alpha$ and $\beta$).

According to the present invention, the isolated and purified active substances can be directly used as a stimulant to stimulate gastrointestinal motility, or, instead, a natural product or a processed natural product containing such active substances may be used. As used herein, the term "natural product" refers to a non-processed grain (for example, barley) or a similar material, and the term "processed natural product" refers to a yeast fermentation product, germinated barley, fractionated product thereof, and a natural product processed in various other manners. The term "fractionated product" as used herein refers to a physically separated product (*i.e.*, tissue fraction). The term "processed natural product" also encompasses directly ingestible compositions obtained by processing natural products, yeast fermentation products, or germinated barley by, for example, making them into fine powders.

Depending on the property required for food and beverage products, additives for use in food and beverage products, pharmaceutical products, animal feeds, and stimulants of gastrointestinal motility of the present invention, the natural product or the processed natural product may be provided as any suitable composition obtained at a desired stage of the extraction, separation, or purification process of the active substances from various natural products or processed natural products. In the present invention, these compositions containing the active substances are also encompassed by the term "processed natural product."

Examples of the yeast fermentation products to serve as the starting material in the present invention include fermentation products of yeast strains of genus *Saccharomyces cerevisiae.* Specific examples of the yeast fermentation products are non-volatile fractions remaining after distillation of whiskeys and other distilled liquors, and yeast-fermented solutions containing yeast fermentation products, such as beer, wine, and rice wine.

For example, the desired fraction that exhibits the property of stimulating gastrointestinal motility via muscarinic $M_3$ receptors can be obtained from the yeast fermentation products as follows: The yeast fermentation products are brought into contact with a synthetic adsorbent (e.g., DIAION HP-20, MICHBISHI CHEMICAL) to allow particular components to adsorb onto the adsorbent, and the adsorbed components are eluted with an alcohol, such as methanol and ethanol, to give the desire fraction capable of stimulating gastrointestinal motility.

If necessary, the resulting fraction may subsequently be exposed to a cation exchange resin (e.g., CM-sephadex C-25, PHARMACIA) at an acidic pH to obtain a cationic component as the fraction of the yeast fermentation product capable of stimulating gastrointestinal motility, which is then purified by HPLC or is crystallized in an organic solvent such as ether to obtain the desired active substances at high purity.

Alternatively, the active substances may be isolated from barley, germinated barley, or fractionated products of germinated barley. The term "barley" as used herein refers to any plant of genus *Hordeum*, including *Hordeum vulgare L.,* and *Hordeum distichon L.* Agronomically, it can be divided into spring barley and winter barley depending on the seeding season. Barley is also divided into two-row barley and six-row barley based on the number of kernel rows. Specific varieties include Haruna nijo, Amagi nijo, Mikamo golden, and Takaho golden, each bred in Japan, and Alexis, Schooner, Harrington, Orbit, Corniche, and Triumph, each bred outside of Japan.

As used herein, the term "germinated barley" refers to grown or developed grains of barley. In the malt production, the term refers to green malts (raw malts) and dried malts. As far as growing barley grains is concerned, the term refers to sprouts and seedlings. During malt production, the degree of sprouting of barley can be determined by controlling factors such as the growth temperature, the amount of water supply, the ratio of oxygen to carbon dioxide in the surface layer, and the length of the sprouting period. Preferably, the water content of green malts (raw malts) is in the range of about 40 to 45% and the water content of dried malts is in the range of about 3 to 15%.

According to the present invention, the fractionated product of germinated barley may be any tissue fraction, including husk, starch layer (albumen), hull and testa, leaf buds, browse, shoot, acrospire, aleurone layer, barley malt sprouts, rootlets, and mixtures thereof. The fractionated products of germinated barley can be prepared by using any known technique, including crushing, sieving, dehusking, air-blowing, selection by specific gravity, and surface removal.

Of the different fractionated products, barley malt sprouts, which are rootlets or roots that grow or sprout during the germination of barley (which may be referred to as "rootlets"), are preferably used in the present invention as the natural product or the processed natural product containing the desired active substances $\alpha$ and $\beta$.

To obtain a desired composition capable of stimulating gastrointestinal motility via muscarinic $M_3$ receptors from the barley, the germinated barley, or the fractionated product of germinated barley, any of known extraction or separation techniques may be used. Specific examples include separation based on partition equilibrium, such as solid-liquid extraction (*e.g.*, aqueous extraction system and organic extraction system), supercritical gas extraction, and adsorption (*e.g.*, activated carbon); separation based on difference in speed, such as filtration, dialysis, and membrane separation (*e.g.*, ultrafiltration, RO, and functional membrane), and liquid chromatography (*e.g.*, ion exchange); separation based on preferential precipitation, such as crystallization, and precipitation by organic solvents. These techniques may be used in proper combination.

If necessary, concentration, filtration, drying, and other necessary process may be carried out to obtain the desired composition for stimulating gastrointestinal motility as a concentrated extract, powder, dried product, crystallized product, or in various other forms. The purity of the composition may be determined depending on the property of desired food and beverage products, additives for use in food and beverage products, pharmaceutical products, animal feeds, and stimulants of gastrointestinal motility: The composition may be provided either as a crude product or as a highly purified product.

A desired composition capable of stimulating gastrointestinal motility via muscarinic $M_3$ receptors can be obtained from the barley malt sprouts and/or rootlets in the following manner: Husks or other contaminants, if any, are removed from the barley malt sprouts and/or rootlets in a pretreatment, such as sieving. When it is desired to process the barley malt sprouts and/or rootlets into a directly ingestible composition, they may be crushed, using a generally known crush technique, into fine powder, or they may be formed into a paste or pellets. When it is desired to extract or separate a composition for stimulating gastrointestinal motility from the barley malt sprouts and/or rootlets, such composition is extracted or separated with water or a proper solvent. If necessary, the purity of the composition may further be increased by exposing the extract to various adsorbents, such as synthetic adsorbent, activated carbon, and ion exchange resin, or subjecting it to a separation/purification technique, such as separation membrane. The extract may be concentrated, filtrated, or dried to form the composition into a concentrated extract, powder, dried product, crystallized product, or various other forms. Alternatively, the extract may be mixed with a sugar to form a syrup-like composition. The composition may be formed into any desired salt, such as salt with hydrochloric acid.

According to the present invention, the barley malt sprouts and/or rootlets may be directly mixed with other materials, and the mixture may be subjected to an extraction/separation process to make a beverage containing the desired stimulant of gastrointestinal motility. For directly ingestible beverages, the amount of the barley malt sprouts and/or rootlets blended in the starting material is preferably in the range of 20mg to 200g per liter of a final product.

The amount of the active substances is preferably in the range of 0.01mg to 100mg per liter of a final product.

The stimulant of gastrointestinal motility acting on muscarinic $M_3$ receptors obtained in the manner described above may be directly added to a food and beverage product as an additive to stimulate gastrointestinal motility, or, if necessary, it may be combined with a proper edible carrier to form a dried product, liquid product, or powder product to serve as an additive. The stimulant may also be formed into a readily soluble formulation that can be dissolved in an alcoholic beverage or mineral water either prior to, or upon use.

According to the present invention, the stimulant of gastrointestinal motility acting on muscarinic $M_3$ receptors may be added to a food and beverage product in the form of the above-defined natural product or processed natural product containing the stimulant, or, if necessary, such a natural product or processed natural product may be combined with a proper edible carrier to form a dried product, liquid product, or powder product to serve as an additive. The natural product or the processed natural product containing the stimulant may also be formed into a readily soluble formulation that can be dissolved in an alcoholic beverage or mineral water either prior to or upon use.

The additives for use in the food and beverage product of the present invention are additives intended for different purposes, including seasonings, liquid seasonings, flavors, furikake (seasoned dried food powder for sprinkling over cooked rice), cooking oils, soup stocks, and supplements, each of which contains the stimulant of gastrointestinal motility as one of its components.

By using a generally known technique, the stimulants and the compositions for stimulating gastrointestinal motility and the mixtures thereof, each of which can serve as an active ingredient of the present invention, may be processed, along with a food material, into food and beverage products, health food products, or specially designed food products.

The food and beverage product according to the present invention may be of any type and form, including solid or liquid products and luxury food products. Examples include agricultural products, such as bread, noodles, cooked rice, sweets (*e.g.*, biscuits, cakes, candies, chocolates, Japanese sweets, gummy candies, and chewing gum), and tofu and processed tofu products; seasonings, such as mirin (Japanese sweet sake for seasoning), vinegar, soy sauce, fermented soybean paste, and dressings; livestock products, such as yogurt, ham, bacon, sausage, and mayonnaise; fish paste products, such as kamaboko (boiled fish cakes) and hanpen (puffy fish cakes); beverages, such as fruit juice-based drinks, soft drinks, sports drinks, coffee-based beverages, tea-based beverages, and carbonated beverages; and milk and powdered milk products, such as powdered milk for babies, and milk for use in coffee.

The health food product according to the present invention may also be of any type and form, including tablets, capsules, and solid or liquid products.

The specially designed food product according to the present invention includes food products designed for sick or weakened people, powdered milk for pregnant women and nursing mothers, artificial powdered milk for babies, food products for aged people, and food with health claims (*e.g.*, food with nutrient function claims, and food for specified health uses).

The active ingredient according to the present invention may be added to fermented malt beverages, such as beer, low malt beer, or low alcohol fermented malt beverages, or to fermented liquors, such as wine, rice wine, or medicinal liquors, to make alcoholic beverages with enhanced stimulation to gastrointestinal motility and drinkability. The active ingredient may also be added to distilled liquors, such as whiskey, brandy, or shochu (Japanese distilled spirit), to make aperitifs/appetizers with ability to stimulate gastrointestinal motility and enhance drinkability.

The present invention also provides a stimulating agent containing the active substances of the present invention for stimulating gastrointestinal motility. Such a stimulating agent may be formed into capsules, tablets, powders, granules, drinkable preparations, syrups, injections, preparations for drip infusion, and other preparations, by using conventional preparation techniques. These preparations may be administered before or during meals to stimulate gastrointestinal motility, or they may be ingested as luxury foods. The stimulating agent can be used not only to stimulate gastrointestinal motility in healthy individuals, but also to recover reduced gastrointestinal motility due to surgical operation, aging, fatigue, diseases, and injuries and to cope with decreased appetite due to chemotherapy.

The stimulants of gastrointestinal motility in accordance with the present invention act on muscarinic $M_3$ receptors. The significant roles played by muscarinic $M_3$ receptors in various physiological activities clearly suggest that the substances of the present invention are capable not only of stimulating gastrointestinal motility, but also of stimulating urination, gastric acid secretion, and appetite.

The stimulants of gastrointestinal motility in accordance with the present invention have also proven to have an ability to enhance drinkability.

Instead of directly using the substances of the chemical formulae (I) through (III) isolated from beer, natural products (*e.g.*, barley) or processed natural products (e.g., yeast fermentation products, germinated barley, and fractionated products of germinated barley) containing such substances may be used as the stimulant of gastrointestinal motility, urination, gastric acid secretion and appetite, as well as the enhancer of drinkability. In particular, barley malt sprouts and processed products of barley malt spouts contain substantial amounts of the substances of the chemical formulae (I) through (III) and are suitably used.

Such natural products and processed natural products may be subjected to extraction/separation processes to obtain particular compositions. The resulting compositions may suitably be used depending on the property of the desired food and beverage products, the additives for use in food and beverage products, the pharmaceutical products, animal feeds, and the stimulating agents of gastrointestinal motility.

When the stimulant of gastrointestinal motility acting on muscarinic $M_3$ receptors according to the present invention is added to food and beverage products for ingestion by humans or other animals, it is added in an amount sufficient to effectively stimulate gastrointestinal motility. For example, the amount of the stimulant added may be such that a few milligrams of the active substance are consumed in every meal. The amount of the stimulant is adjusted so that preferably from 0.01mg to 100mg, more preferably from 0.05mg to 50mg, and even more preferably from 0.1mg to 10mg of the active substance is ingested everyday, although the amount may vary depending on the form of the food and beverage products, additives for use in food and beverage products, stimulants of gastrointestinal motility, and animal feeds.

It was demonstrated that the stimulant of gastrointestinal motility according to the present invention did not cause death or bring about any anomalies in general conditions or body weight when as much as 50mg/kg was orally administered to mice in an acute toxicity test. The stimulant has thus proven to have little or no toxicity.

As set forth, the present invention is effective in stimulating gastrointestinal motility and enhancing drinkability and can thus be applied to subjects who suffer from anorexia, dyspepsia, feeling of fullness after meals, heavy stomach feeling, nausea, vomiting, epigastralgia, and other gastrointestinal symptoms caused by reduced gastrointestinal motility.

## Examples

The present invention will now be described in further detail with reference to Experiments and Examples, which are provided by way of example only and are not intended to limit the scope of the invention in any way.

Studies have shown that dried beer products stimulate gastrointestinal motility by acting on muscarinic $M_3$ receptors. Thus, a search was conducted for stimulants of gastrointestinal motility present in beer based on the binding activity to muscarinic $M_3$ receptors.

### Reference Example 1: Test for binding activity to muscarinic $M_3$ receptors

A test was conducted for the binding activity to muscarinic $M_3$ receptors in the following manner.

A specimen (a dried beer product and a fractionated product of a dried beer product) and 0.2nM [$^3$H]4-diphenylacetoxy-N-methylpiperidine methiodide ([$^3$H]4-DAMP) to serve as a ligand were added to a suspension of a cell membrane preparation obtained from a transfected Chinese hamster ovary (CHO) cell line expressing human muscarinic $M_3$ receptors. The mixture was then incubated at 22°C for 60min. Subsequently, the reaction mixture was filtered through a glass fiber filter (GF/B, PACKARD) while aspirated. Once the reaction came to an end, the mixture was washed several times with ice-chilled buffer. A scintillation cocktail (Microscint O, PACKARD) was then added to the filter, and the residual radioactivity was measured by a liquid scintillation counter (Topcount, PACKARD). The amount of specific binding of [$^3$H]4-DAMP was determined by subtracting the amount of non-specific binding in the presence of 1μM atropine from the amount of total binding of [$^3$H]4-DAMP.

The inhibition curve of [$^3$H]4-DAMP binding by the dried, beer product is shown in Fig. 1. As can be seen from the results of Fig. 1, the dried beer product inhibited binding of [$^3$H]4-DAMP at concentrations higher than 0.1mg/mL, and its $IC_{50}$ value (the inhibitor concentration at which 50% of the [$^3$H]4-DAMP binding is inhibited) was approximately 2mg/mL.

### Example 1:

In order to isolate stimulants of gastrointestinal motility from beer, fractions exhibiting an inhibitory activity against the ligand binding were separated/purified by using, as the standard, the binding activity of the ligand to muscarinic $M_3$ receptors obtained in Reference Example 1. The flow of the separation/purification process is shown in Fig. 2.

Specifically, 10L beer (100% malt, 5% alcohol, dry weight = 3.5%w/v) was freeze-dried. To this dried product, pure water was added and the solution was subjected to a chromatography using a synthetic adsorbent (XAD-2). The adsorbed material was eluted with methanol to give an eluate showing a binding activity to muscarinic $M_3$ receptors. From the eluate, methanol was evaporated and pure water was added to the dried product. The solution was then subjected to a weakly acidic, cation exchange chromatography (resin: CM-Sephadex C-25). The adsorbed material was eluted with methanol/hydrochloric acid to give an eluate showing a binding activity. From the eluate, methanol and hydrochloric acid were evaporated and pure water was added to the dried product. The solution was then subjected to a gel filtration chromatography (resin: Sephadex G-15). Subsequently, fractions exhibiting a binding activity (Active

fraction No. 1: Kd = 3-5; active fraction No. 2: Kd = 7-9) were each subjected to a preparative ODS-HPLC. As a result, 4.2mg of the active substance α were obtained from the active fraction No. 1 and 12.6mg of the active substance β were obtained from the active fraction No. 2.

The two active substances (α and β) obtained above were subjected to HPLC analysis. The HPLC analysis chart of the active substance α is shown in Fig. 3.

As indicated by the single peak shown in the figure, the active substance α was eluted at the retention time of about 33 to 34min.

Similarly, the active substance β was eluted at substantially the same retention time as the active substance α and was confirmed by the single peak on HPLC.

Also, the active substances α and β were each shown to give a single peak when subjected to HPLC analysis under different conditions.

Using respective doses of the active substances α and β, each of which was eluted to give a single peak on HPLC, a dose-response curve was constructed for each substance by conducting tests for binding activity to muscarinic $M_3$ receptors as in Reference Example 1.

The results are shown in Fig. 4 (the active substance α) and Fig. 5 (the active substance β), respectively. As can be seen from the results shown in these figures, the active substances α and β each show a dose-dependency and have respective $IC_{50}$ values of 0.65 x $10^{-6}$g/mL and 2.30 x $10^{-6}$g/mL.

4-DAMP had an $IC_{50}$ value of 0.62 x $10^{-9}$g/mL.

In summary, two active substances (*i.e.*, active substances α and β) were isolated from beer and each was shown to give a single peak on HPLC and act on muscarinic $M_3$ receptors in a dose-dependent manner.

<u>Example 2:</u>

The two active substances (α and β) isolated/purified above were subjected to analysis by UV spectrophotometry, mass spectrometry, and NMR spectrometry.

1. UV spectrophotometry

The results of UV spectrometry performed on the substances α and β are shown in Fig. 6.

The two substances showed very similar UV spectra: a single absorption peak around 230nm and a relatively broad absorption peak around 300nm, the pattern unique to these substances.

2. Mass spectrometry (FAB-MASS)

Mass spectrometry (FAB-MASS) analysis was also conducted on the active substances α and β.

The active substances α and β were individually dissolved in 3-nitrobenzyl alcohol and were subjected to mass spectrometry. The results are shown in Fig. 7 (active substance α) and Fig. 8 (active substance β). As shown, each active substance generated molecular ions $(M+H)^+$ at a mass-to-charge ratio (m/z) of 551 and each was shown to have a molecular weight of 550.

3. NMR ($^1$H-NMR, $^{13}$C-NMR) spectrometry

NMR ($^1$H-NMR, $^{13}$C-NMR) spectrometry analysis was conducted on the active substances α and β. Specifically, the NMR analysis was conducted on DMX-750 ($^1$H-NMR) and DMX-500 ($^{13}$C-NMR) (BRUKER BIOSPIN) using DMSO-$D_6$ solvent.

The results of $^1$H-NMR spectrometry are shown in Fig. 9 (active substance α) and Fig. 10 (active substance β), and the results of $^{13}$C-NMR spectrometry are shown in Fig. 11 (active substance α) and Fig. 12 (active substance β).

The $^1$H-NMR and $^{13}$C-NMR spectra of the active substances α and β were similar in the number of signals and showed very similar chemical shifts and signal split patterns, indicating a high structural similarity between the two compounds.

4. Structure determination

As described above, the results of UV spectrophotometry, mass spectrometry, and NMR spectrometry analyses of the isolated active substances α and β indicate that the two compounds are stereoisomers of the compound shown by the following chemical formula:

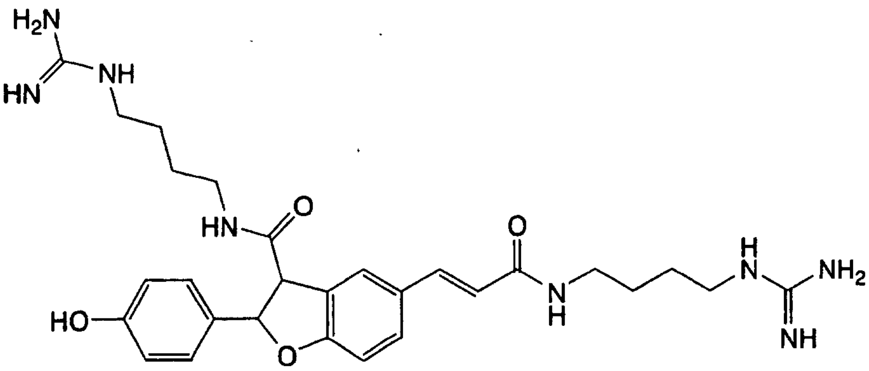

(I)

The structural formulae of the active substances α and β have been shown to be represented by the following chemical formulae (II) and (III), respectively:

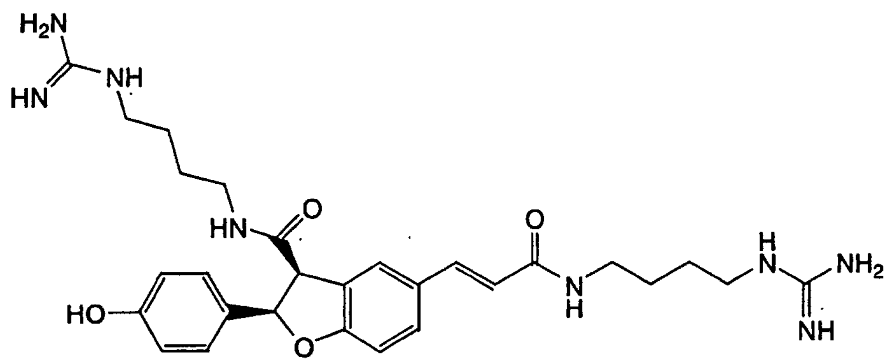

(II)

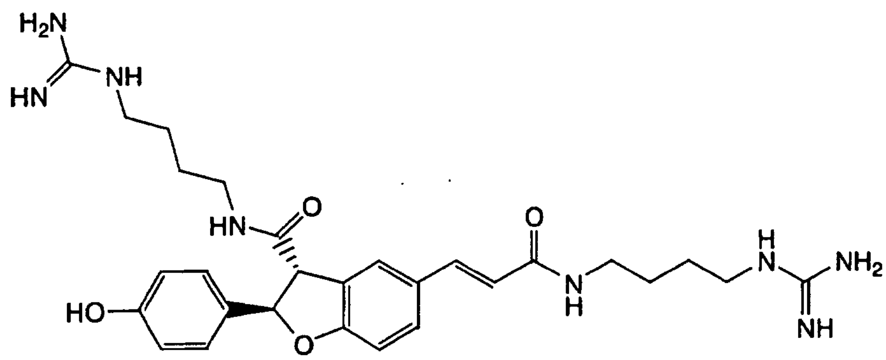

(III)

The absolute configuration of each stereoisomer was determined by CD curve analysis. The CD spectra of the active substances α and β were obtained on a JASCO J-725 spectropolarimeter using methanol solvent.

The active substances α and β of the present invention have been shown to have absolute configurations that give the CD spectra shown in Figs. 13 and 14, respectively.

<u>Example 3:</u>

The isolated/identified active substances α and β were evaluated for their ability to stimulate gastrointestinal motility based on their activity to stimulate stomach emptying in mice.

1. Method

Using a stomach tube, ddY line mice (10 animals in each group) were orally administered each of distilled

water, the active substances α and β following an 18-hour fasting period. 60 minutes after administration, each animal was orally administered a test feed (0.2mL/animal; 1.5% methylcellulose solution containing 0.05% phenol red) using the same technique. After 15 minutes, the animals were sacrificed by cervical dislocation and the stomachs were removed. The stomachs, along with their contents, were then homogenized in 10mL 0.1N NaOH and the homogenates were allowed to stand for 1 hour at room temperature. To 1mL of the resulting supernatant, 0.1mL 20% trichloroacetic acid was added and the supernatant was centrifuged to precipitate proteins. Subsequently, 0.5ml of the resulting supernatant was collected and 0.5mL 0.5N NaOH was added to color the solution. The absorbance of the solution at 560nm was measured to determine the amount of phenol red remaining in the stomach. The rate of stomach emptying was determined by the following equation:

$$\text{Stomach emptying rate (\%)} = (A-B)/A \times 100$$

where 'A' is the amount of phenol red in the stomach immediately after the administration of the test feed; and 'B' is the amount of phenol red remaining in the stomach 15 minutes after the administration of the test feed.

2. Results

As shown in Fig. 15, the results for each group were expressed in means and standard errors, which were tested for significant differences using Student t-test.
As can be seen from the results shown in the figure, each of the active substance α (0.1 and 0.3mg/kg) and the active substance β (0.3 and 1.0mg/kg) significantly stimulated stomach emptying in a dose-dependent manner ($P<0.05$), indicating that each substance has a strong ability to stimulate gastrointestinal motility.
The significant roles played by muscarinic $M_3$ receptors in various physiological activities clearly indicate that each of the present substances is capable not only of stimulating gastrointestinal motility, but also of stimulating urination, gastric acid secretion and appetite.

Example 4:

A variety of naturally-occurring materials that are rich sources of the active substances α and β were subjected to the same purification process and HPLC analysis as in Example 1 to obtain compositions containing the native active substances.
Methanol extracts of each material were freeze-dried and were then dissolved in pure water. Each solution was subjected to weakly acidic, cation exchange chromatography and gel filtration chromatography, and the resulting fractions (Kd = 3-5, and Kd = 7-9) were analyzed by HPLC in the same manner as in Example 1. The presence of the active substances and their concentrations were determined from the retention time and the peak area.
As a result, barley malt sprouts, one of the fractionated products of germinated barley, were found to contain the active substances.
Shown in Fig. 16 is an HPLC analysis chart of a fraction (Kd = 7-9) obtained from the extract of barley malt sprouts.
As indicated by an arrow in the figure, the active substance was detected as a single peak at a retention time of 33 to 34 minutes.
By adding the active substance β isolated in Example 1 to this fraction (Kd = 7-9) and subjecting the solution to HPLC analysis, it was confirmed that the resulting peaks overlap and the active substance β is present in barley malt spouts. Similarly, the fraction with a Kd of 3 to 5 was analyzed to confirm the presence of the active substance α in barley malt sprouts. Also, the extract of barley malt sprouts was subjected to a purification process by HPLC to isolate the active substances α and β, which were then subjected to UV spectrophotometry, mass spectrometry, and NMR spectrometry to confirm that the spectra were consistent with the spectra obtained in Example 2.
The concentrations of the respective active substances in barley malt sprouts were also determined from the area of the respective peaks, and it was found that the barley malt sprouts contain the active substances α and β at high concentrations (approximately 0.05mg and 0.5mg per 1g of barley malt sprouts, respectively).
The fact that barley malt sprouts, one of the fractionated products of germinated barley, contain the active substances of the present invention implies the presence of the active substances in other components of the fractionated products of germinated barley, including husks, starch layer (albumen), hull and testa, leaf buds, acrospire, aleurone layer, and rootlets, as well as in barley and germinated barley.
The above discussion confirms that a variety of compositions/additives containing the native active substances (*i.e.*, active substances α and β) can be obtained by subjecting barley malt sprouts and related materials (*e.g.*, fractionated products of germinated barley, barley, and germinated barley) to an extraction/separation process as described above.

Example 5:

In order to determine if the extract of barley malt sprouts, which contains the active substances, has an ability to stimulate gastrointestinal motility, the effects of the extract on the contraction of longitudinal muscles of guinea pig ilea were examined according to Magnus' method.

Specifically, a length of longitudinal muscle collected from the ileum of male guinea pigs of the Hartley line was suspended from a transducer while immersed in a Krebs-Ringer solution. The solution was maintained at 35°C and was saturated with a 95% $O_2$/5% $CO_2$ gas for stabilization. This setup was prepared for each of the following materials: a dried product of an extract of barley malt sprouts to serve as a test product ($IC_{50}$ = 0.20x$10^{-3}$g/mL, where $IC_{50}$ is defined as the inhibitor concentration at which 50% of binding of the standard ligand to $M_3$ receptors is inhibited (See, Reference Example 1)); a dried product of beer to serve as a control ($IC_{50}$ = 2.00x$10^{-3}$g/mL, $IC_{50}$ as defined in Reference Example 1); and carbachol to serve as a positive control. The movement of the longitudinal muscle in each setup was recorded, and once the constant voluntary movement was achieved, each of the test product, control, and positive control was applied to the muscle in each setup and contraction of each muscle was measured.

The results are shown in Fig. 17. As can be seen from the figure, the dried product of the barley malt sprout extract elicited contraction of the longitudinal muscles of guinea pig ilea in a dose-dependent manner in the concentration range of 0.005 to 0.5mg/mL. Assuming the contraction elicited by carbachol (5x$10^{-3}$M) to be 100, the dried product of the barley malt spout extract showed an activity approximately 20 times as high as that of the dried beer product as the control.

These observations demonstrate that the barley malt extract containing the active substance has a strong ability to stimulate gastrointestinal motility.

Example 6: Sensory test in humans

In this example, a beverage (an alcoholic beverage) is prepared as an example of the food and beverage product of the present invention containing a composition to stimulate gastrointestinal motility, the composition obtained from a natural product or a processed natural product containing the active substances of the present invention (*i.e.*, active substances α and β). The beverage is evaluated for its ability to stimulate gastrointestinal motility and urination.

1. Preparation of alcoholic beverage

In Example 4, we have demonstrated that the barley malt spouts contain the native active substances (*i.e.*, active substances α and β). Based on this finding, an alcoholic beverage was prepared containing a particular composition, obtained as an aqueous extract of barley malt sprouts (a water-soluble fraction of barley malt sprouts). The composition used was approximately 30g of a dried product prepared by crushing approximately 150g of barley malt sprouts, followed by extraction with pure water and filtration (approx. 20% yield (w/w); equivalent to approx. 22.5mg of the active substance α and approx. 75mg of the active substance β).

A trial product 1 as a plum-flavored alcohol beverage with 5% alcohol content was prepared in the following manner: 850mL 59% alcohol, 100mL clear plum juice concentrated 5 times, 200g sugar, 20g citric acid, 6ml flavor, and 30g of the composition described above (*i.e.*, aqueous barley malt sprout extract) were mixed together and pure water was added to a final volume of 10L. This mixture was heat-sterilized, and after cooling, pressurized carbon dioxide was added. The mixture was then filled in a 250mL can and the can was sealed. The final product contained the active substances α and β in amounts of approximately 2.25mg/L and 7.5mg/L, respectively.

A similar alcohol beverage without the composition was also prepared and was assigned as a control product 1.

2. Sensory test

The trial product 1 and the control product 1 were evaluated for their ability to stimulate gastrointestinal motility and urination in human subjects. The evaluation was made in the following manner.

Twenty healthy male subjects were divided into two groups of ten. One group was assigned to the trial product 1 and the other to the control product 1. Tests were conducted according to the double blind method. The two groups were switched around after a two-week interval.

The subjects were allowed to consume the trial product 1 or the control product 1 for 2 hours at a rate of 2.4mL/kg per every 15min. The subjects were also allowed to consume potato chips for 2 hours at a rate of 0.2g/kg per every 15min.

To evaluate the ability of each product to stimulate gastrointestinal motility, the subjects were asked to rate the degree of stomach distension after 60 and 120 minutes and write down the rating.

3. Evaluation criteria

1) The degree of stomach distension was rated on the following scale:

| | |
|---|---|
| stomach felt distended | 3 points |
| stomach felt somewhat distended | 2 points |
| no or little sense of distension | 1 point |

2) To evaluate the ability of each product to stimulate urination, the subjects were allowed to urinate prior to the test and were asked to go to the bathroom every 30 minutes during the test and the urine volume was measured.

4. Results

The results of the evaluation of the sense of stomach distention are shown in Table 1 below.

Table 1

| | The number of responded subjects in the rating of the sense of stomach distension | | | |
|---|---|---|---|---|
| | Trial product 1 | | Control product 1 | |
| Responses of 20 subjects | 60min. | 120min. | 60min. | 120min. |
| Significant sense of stomach distension (3pts) | 0 | 3 | 2 | 15 |
| Minor sense of stomach distension (2pts) | 3 | 16 | 16 | 5 |
| Little or no sense of stomach distension (1pt) | 17 | 1 | 2 | 0 |
| Average | 1.2 pts | 2.1 pts | 2.0 pts | 2.8 pts |

As can be seen from the results above, many of the subjects who ingested the trial product 1 felt little or no stomach distension after 60 minutes, and few felt significant stomach distension and many felt only minor stomach distention even after 120 minutes.
In comparison, many of the subjects who were administered the control product 1 started to suffer minor stomach distension after 60 minutes and felt significant stomach distension after 120 minutes.
These results indicate that the trial product 1, which contains the composition containing the active substances of the present invention, has a significant ability to suppress the degree of the sense of stomach distention and stimulate gastrointestinal motility.
Fig. 18 shows the results of the measurement of urine volume after 30, 60, 90, and 120 minutes (in mean values).
The results indicate that the urine volume was significantly greater in the group administered the trial product 1 than in the group administered the control product 1 at the respective time points.
This proves that the substances of the present invention has not only an ability to stimulate gastrointestinal motility, but also an ability to stimulate urination, as is implied by the involvement of muscarinic $M_3$ receptors in various physiological activities.

Example 7:

Given that the active substances (*i.e.*, active substances $\alpha$ and $\beta$) have an ability to stimulate gastrointestinal motility and urination, we examined the active substances for their ability to enhance drinkability in rats.

1. Method:

Specifically, a non-alcoholic beverage was prepared by adding the same composition as in Example 6 (water extract composition of barley malt sprouts) to a 3% aqueous solution of sugar to the same final concentration as in Example 6 (3g/L water extract of barley malt sprout). This beverage was assigned as a trial product 2 (the final product contained approx. 2.25mg of the active substance $\alpha$ and approx. 7.5mg of the active substance $\beta$). Another non-alcoholic beverage was prepared as a 3% aqueous solution of sugar without the active substances and was assigned as a control product 2.

Two groups of rats (3 animals in each group) were individually allowed to freely feed on each of the trial product 2 and the control product 2, and the amount of ingested water was measured over 5 days.

2. Results

Fig. 19 shows the mean value of the amount of water ingested by each rat fed with the trial product 2 or the control product 2 during each period in which evaluation was made (i.e., Day 1 to Day 2, Day 2 to Day 3, Day 3 to Day 4, and Day 4 to Day 5).

As can be seen from the results, water consumption is significantly larger in the group fed with the trial product 2 than in the group fed with the control product 2 in each of the evaluation periods.

These results demonstrate that the active substances of the present invention have, in addition to the ability to stimulate gastrointestinal motility, an ability to enhance drinkability. Not only does this ability of the active substances make it possible for the beverages to which the substances are added to be drinkable in substantial quantities and still remain refreshing, but it also affects the refreshingness of the beverages itself. In this example, it was demonstrated that the active substances of the present invention can impart such favorable properties to the beverages to which they are added.

Example 8: Comparison of ingestion behavior of the food and beverage products in humans

A beer was produced as an example of fermented liquor containing the composition of the present invention (*i.e.*, water extract composition of barley malt sprouts) containing the active substances, and its ingestion behavior was observed in human subjects.

Using malt, hops, the composition containing the active substances (*i.e.*, water extract composition of barley malt sprouts), and water as starting materials, the beer was produced according to an ordinary technique: 100L of the ingredients were prepared and were subjected to fermentation and filtration. The resulting product was filled in a container. More specifically, 15kg crushed malt was processed in a tun to convert the starch into sugar and the above-described composition (0.4kg, equivalent to 2kg barley malt sprouts) was added as a sub-material. The resulting material was filtered in a filtration vessel. Subsequently, hops were added and the resulting material was boiled in a boiling vessel. The resultant sediment was removed in a sedimentation vessel, known as a whirl pool. To the resulting clear solution, yeast was added and the solution was fermented and stored at -1° C.

Subsequently, the fermented solution was filtered to remove yeast. This gave a fermented liquor with approximately 4% alcohol content (assigned as trial product 3. The final product contained the active substances $\alpha$ and $\beta$ in amounts of approx. 3.0mg/L and approx. 10.0mg/L, respectively). A similar beer without barley malt sprouts was also prepared to serve as a control (assigned as control product 3).

The comparison of ingestion behavior of the food and beverage product was performed according to crossover trial in conjunction with double blind method. Specifically, 20 subjects were divided into two groups, and each group was allowed to freely consume each of the beer with barley malt sprouts and the beer without barley malt sprouts while eating a particular meal as much as they want over a two-hour period. The change in the amount of the beer consumption was monitored and the amount of leftover meal was measured after 2 hours. Specifically, the served meal consisted of the following items: various appetizers, tofu and sashimi at 0min., meat steaks at 30min., grilled egg plant and fried tofu at 60min., and rice, soup, and fruits at 90min. The amount of beer consumption was measured every 30 minutes for 120 minutes.

The mean values of the amounts of beer consumption are shown in Fig. 20. The results show that the beer to which the composition (*i.e.*, water extract composition of barley malt extract) containing the active substances have been added (Trial product 3) tends to be consumed in larger amounts than the beer without such a composition (Control product 3), indicating the greater drinkability of the trial product 3.

The amounts of the meal left after 2 hours are shown in Table 2 below.

Table 2

| | Average amounts of leftover meal for 20 subjects | |
|---|---|---|
| | Ingested trial product 3 | Ingested control product 3 |
| Rice | 21g | 145g |
| Soup | 5ml | 120ml |
| Fruits | 3g | 35g |

As can be seen from the results, the amount of the leftover meal is significantly smaller in the group ingesting the trial product 3 than in the group ingesting the control product 3. This implies that the trial product 3 has an ability to stimulate appetite.

These observations collectively indicate that the beverages containing the composition containing the active substances have an ability to enhance drinkability and stimulate appetite in humans.

The present invention is now described with reference to examples in which beverages and precursors of pharmaceutical products containing the active substances of the present invention are examined.

Example 9: Production of soft drinks

As a type of a soft drink, a fruit juice-based, "near-water" product was produced according to an ordinary method: A fruit juice, citric acid, sugar, flavors, and the composition containing the active substances (*i.e.*, water extract composition of barley malt sprouts) were mixed together to give a final volume of 1000mL. The mixture was sterilized and was then filled in a container. More specifically, 40g of granulated sugar were weighed and were dissolved in pure water at 50°C. To this solution, various concentrated thick juices (*e.g.*, orange juice (5-fold conc.) and apple juice (4-fold conc.)) were added along with 0.15g citric acid, 3g of the composition of the present invention, 2mL flavor, and 0.25g L-ascorbic acid, followed by addition of water to adjust the mixture to a final volume of 1000mL(1% as measured by non-concentrated juices). The resulting mixture was pasteurized at 100°C for 20 minutes, and 100ml aliquots were filled in 110ml clear bottles and the bottles sealed to give near-water products containing various fruit juices (1% fruit juice; the final product contained the active substances $\alpha$ and $\beta$ in amounts of approx. 2.25mg/L and approx. 7.5mg/L, respectively)

Example 10: Production of tea-based beverage

0.3L of an extract of green tea leaves, 0.3g sodium bicarbonate, and the composition containing the active substances (*i.e.*, water extract composition of barley malt sprouts) were mixed together and pure water was added to adjust the solution to a total volume of 1L. The solution was then heated to 85°C and was filled in a 190g can. The solution was then sterilized in a retort (125°C, 90min.) to make a canned beverage (The final product contained the active substances $\alpha$ and $\beta$ in amounts of approx. 0.25mL and approx. 0.75mL, respectively).

Example 11: Health food

Approximately 1.5kg of barley malt sprouts were subjected to extraction with pure water and the extract was purified by a cation exchange chromatography and ODS-HPLC to obtain approximately 3g of a crude product (approx. 0.2% yield (w/w). The product contained the active substances $\alpha$ and $\beta$ in amounts of approx. 250mg and approx. 750mg, respectively).

Subsequently, 50mg of the crude product, 120g lactose, and 30mg crystallized cellulose were mixed together to make capsuled health food capsules, each capsule containing 200mg content (The final product contained the active substances $\alpha$ and $\beta$ in amounts of approx. 4.17mg/capsule and approx. 12.5mg/capsule, respectively).

Example 12: Precursor of pharmaceutical product

Approximately 150kg of barley malt sprouts were subjected to extraction with pure water and the extract was purified by a cation exchange chromatography, gel filtration chromatography, and ODS-HPLC and was dried to form a solid product. The dried product was dissolved in distilled water and the solution was sterilized, was filtered, and was then ultrafiltered to obtain approximately 45g of a purified product of the active substance $\beta$ at a purity of approx. 99% (as determined by HPLC analysis).

INDUSTRIAL APPLICABILITY

As set forth, the present invention provides a substance that is isolated from beer and has an ability to stimulate gastrointestinal motility by acting on muscarinic M3 receptors, and/or a substance that has an ability to enhance drinkability. Such a substance can be obtained in the form of a composition or a purified product from a natural product or a processed natural product containing such a substance. Such a compound or a purified product can then be used as an additive to food and beverage products to impart to the products the ability to stimulate gastrointestinal motility.

The substance of the present invention capable of acting on muscarinic $M_3$ receptors to stimulate gastrointestinal motility can be used to effectively stimulate gastrointestinal motility in subjects suffering from anorexia, dyspepsia, feeling of fullness after meals, heavy stomach feeling, nausea, vomiting, epigastralgia, and other gastrointestinal symptoms caused by reduced gastrointestinal motility. The ability to enhance drinkability can stimulate appetite as well as drinkability.

Moreover, the composition containing the substance capable of stimulating gastrointestinal motility and/or enhancing drinkability can be used to provide various beverages and functional food products. Thus, such a composition is of significant usefulness.

## Claims

**1.** A compound represented by the following chemical formula (I):

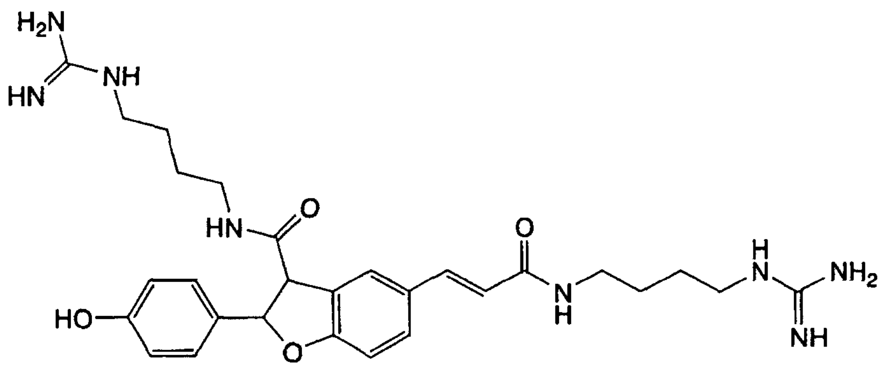

(I)

**2.** A compound represented by the following chemical formula (II):

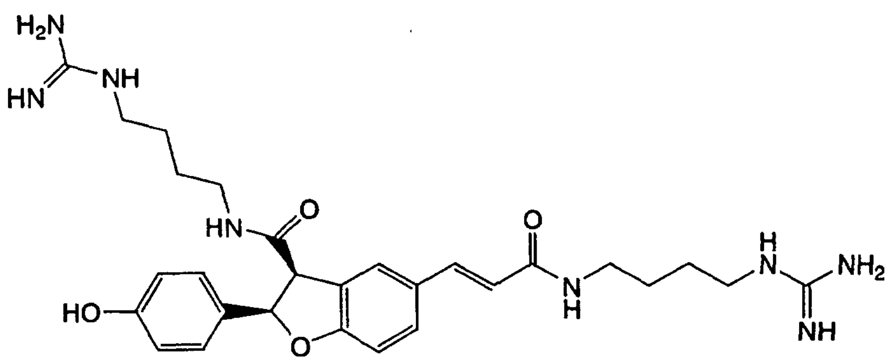

(II)

**3.** A compound represented by the following chemical formula (III):

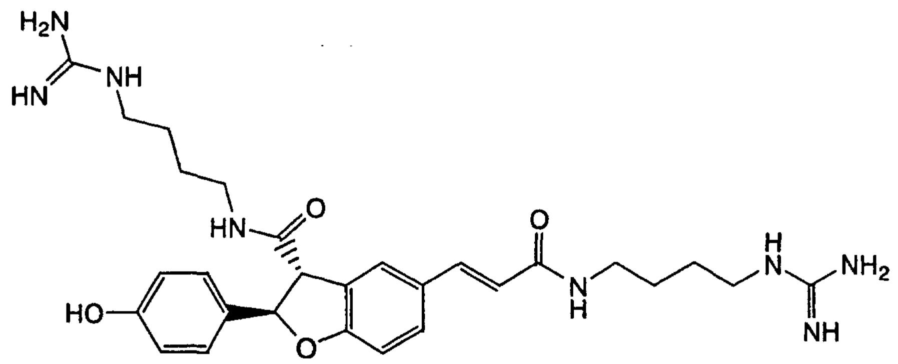

(III)

4. The compound according to claim 1, 2, or 3 isolated from a fermentation product of yeast, barley, germinated barley, or a fractionated product of germinated barley.

5. The compound according to claim 4, wherein the fractionated product of germinated barley is barley malt spouts.

6. The compound according to any one of claims 1 through 5 showing its activity by acting on muscarinic $M_3$ receptors.

7. The compound according to claim 6, wherein the activity of the compound elicited via muscarinic $M_3$ receptors is an ability to stimulate gastrointestinal motility, gastric acid secretion, urination or appetite, or to enhance drinkability.

8. A food or beverage product, an additive to a food or beverage product, a pharmaceutical product, or an animal feed, **characterized by** containing a natural product or a processed natural product containing the compound according to any one of claims 1 through 7.

9. The food or beverage product, the additive to a food or beverage product, the pharmaceutical product, or the animal feed according to claim 8, wherein the natural product or the processed natural product containing the compound according to any one of claims 1 through 7 is a fermentation product of yeast, barley, germinated barley, or a fractionated product of germinated barley.

10. The food or beverage product, the additive to a food or beverage product, the pharmaceutical product, or the animal feed according to claim 8, wherein the natural product or the processed natural product containing the compound according to any one of claims 1 through 7 is barley malt or a processed product of barley malt sprout.

11. A composition showing its activity via muscarinic $M_3$ receptors, **characterized by** containing the compound according to any one of claims 1 to 7, or a natural product or a processed natural product containing the compound.

12. The composition according to claim 11, wherein the activity elicited via muscarinic $M_3$ receptors is an ability to stimulate gastrointestinal motility, gastric acid secretion, urination or appetite, or to enhance drinkability.

13. The composition according to claim 11 capable of stimulating gastrointestinal motility and/or enhancing drinkability.

14. The composition according to any one of claims 11 to 13, wherein the natural product or the processed natural product containing the compound according to any one of claims 1 to 7 is a fermentation product of yeast, barley, germinated barley, or a fractionated product of germinated barley.

15. The composition according to any one of claims 11 to 13, wherein the natural product or the processed natural product containing the compound according to any one of claims 1 to 7 is barley malt spout or a processed product of barley malt sprout.

16. A food or beverage product, containing the compound according to any one of claims 1 to 7, a natural product or a processed natural product, or a composition containing the compound.

17. The food or beverage product according to claim 16, being an alcoholic beverage or a non-alcoholic beverage.

**18.** The food or beverage product according to claim 16, being a fermented malt beverage.

**19.** The food or beverage product according to claim 18, wherein the fermented malt beverage is a beer, a low malt beer or a low alcohol fermented malt beverage.

**20.** The food or beverage product according to claim 18, being a tea-based beverage.

**21.** The food or beverage product according to claims 16 to 20, wherein the final product of the food or beverage product contains the compound of any one of claims 1 to 7 in an amount of 0.01mg/L to 100mg/L.

**22.** The food or beverage product according to claim 16, being a health food and/or a specially designed food product.

**23.** An additive to a food or beverage product, or a pharmaceutical product, containing the compound according to any one of claims 1 to 7, a natural product, a processed natural product, or a composition containing the compound.

**24.** The additive to a food or beverage product, or a pharmaceutical product according to claim 23, capable of acting on muscarinic $M_3$ receptors.

**25.** The use of the compound capable of acting on muscarinic $M_3$ receptors according to any one of claims 1 through 7, a natural product, a processed natural product or a composition containing the compound, in the production of a food or beverage product, an additive to a food or beverage product, a pharmaceutical product, or an animal feed that has an ability to stimulate gastrointestinal motility, gastric acid secretion, urination or appetite, or an ability to enhance drinkability, or in the production of a stimulating agent of gastrointestinal motility or an enhancing agent of drinkability.

**26.** The use according to claim 25, **characterized in that** the natural product or the processed natural product containing the substance having an ability to stimulate gastrointestinal motility via muscarinic $M_3$ receptors is a fermentation product of yeast, barley, germinated barley, a fractionated product of germinated barley, or barley malt sprout.

**27.** A method for separating/isolating the compound according to any one of claims 1 to 3, comprising the steps of:

preparing a natural product or a processed natural product containing the compound according to any one of claims 1 to 7 to serve as a starting material;
removing volatile components through an extraction/solid liquid separation/drying process when the starting material is a solid product and through a drying process when the starting material is a liquid product, thereby collecting non-volatile components;
subjecting the non-volatile components to a chromatography using a hydrophobic adsorbent (*e.g.*, synthetic adsorbent) to remove unadsorbed components and collect adsorbed components as a crude fraction containing the concentrated active substance;
subjecting the crude fraction to a cation exchange chromatography (*e.g.*, weakly acidic cation exchange resin) to remove unadsorbed components;
eluting the active substance with an acidic methanol (methanol/hydrochloric acid);
subjecting the eluate to a gel filtration chromatography using 0.01N hydrochloric acid to obtain a purified fraction; and
subjecting the purified fraction to ODS-HPLC ($C_{18}$-high performance liquid chromatography) for further purification.

**28.** The method according to claim 27, **characterized in that** the starting material is a fermentation product of yeast, barley, germinated barley, a fractionated product of germinated barley, or barley malt sprout.

**29.** The compound according to claim 2, showing the CD spectrum of Fig. 13.

**30.** The compound according to claim 3, showing the CD spectrum of Fig. 14.

Fig. 1

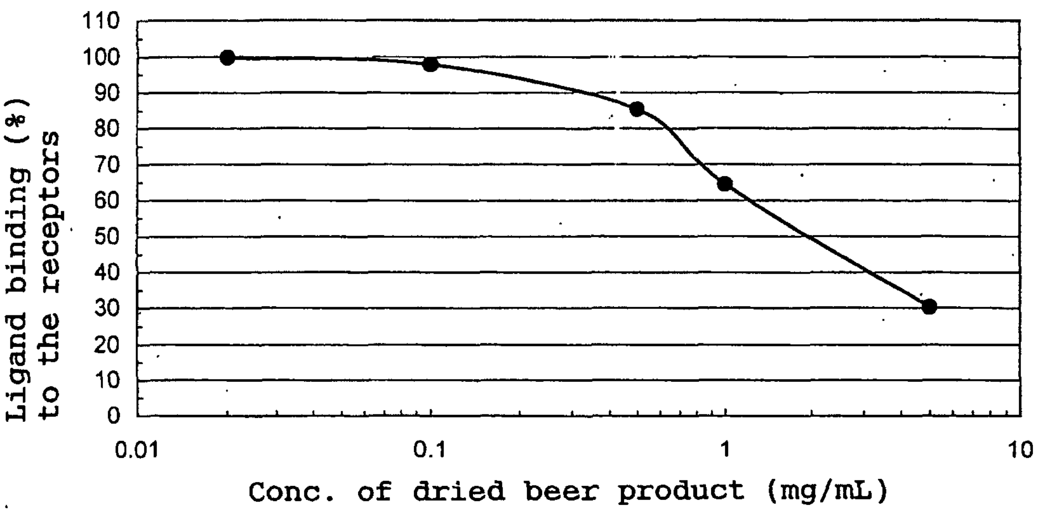
Ligand binding (%) to the receptors
110
100
90
80
70
60
50
40
30
20
10
0
0.01
0.1
1
10
Conc. of dried beer product (mg/mL)

Fig. 2

The numbers in parentheses are amount/yield

Beer (10L)

↓ freeze-drying

Freeze-dried beer (350g)

↓ Chromatography using synthetic adsorbent (adsorbent: XAD-2)

Adsorbed components (methanol elute)(10.9g)

↓ Weakly acidic cation exchange chromatography (resin: CM Sephadex C-25)

Adsorbed components (methanol/HCl elute)(2.19g)

↓ Gel Filtration Chromatpgraphy (resin: Sephadex G-15)

- Active fraction No.1: Kd=3 to 5 (55.5mg)
- Active fraction No.2: Kd=7 to 9 (33.1mg)

↓ Preparative ODS-HPLC

- Active fraction 1: Active substance α (4.2mg)
- Active fraction 2: Active substance β (12.6mg)

Fig. 3

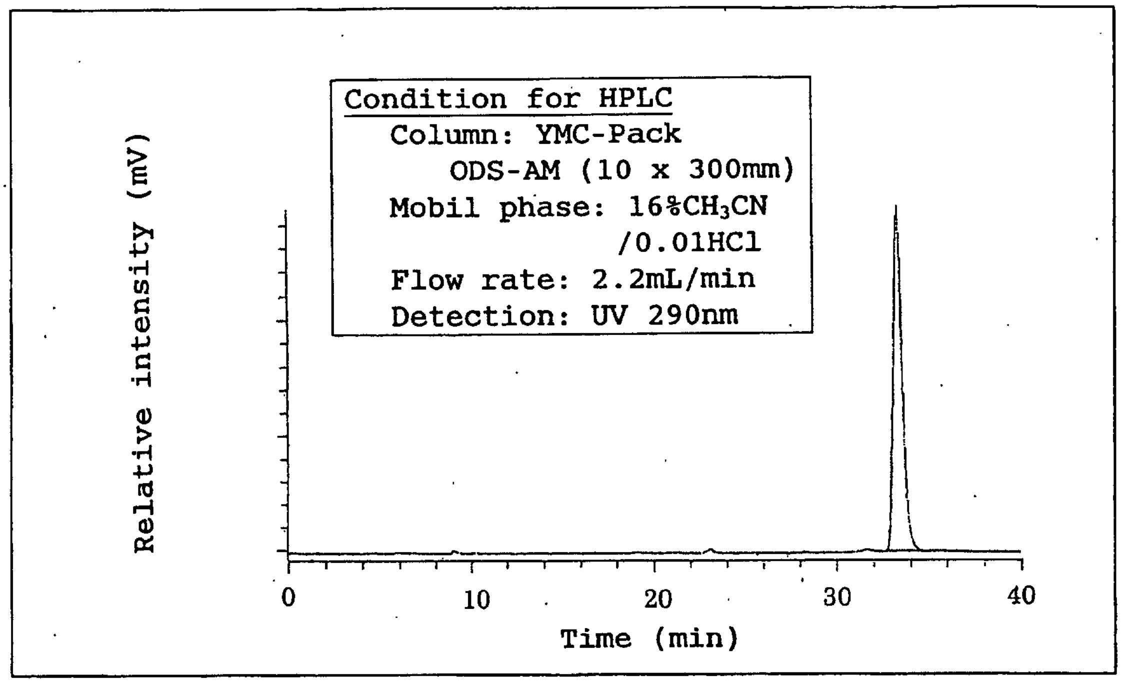
Condition for HPLC
Column: YMC-Pack
ODS-AM (10 x 300mm)
Mobil phase: 16%CH3CN
/0.01HCl
Flow rate: 2.2mL/min
Detection: UV 290nm
Relative intensity (mV)
0
10
20
30
40
Time (min)

Fig. 4

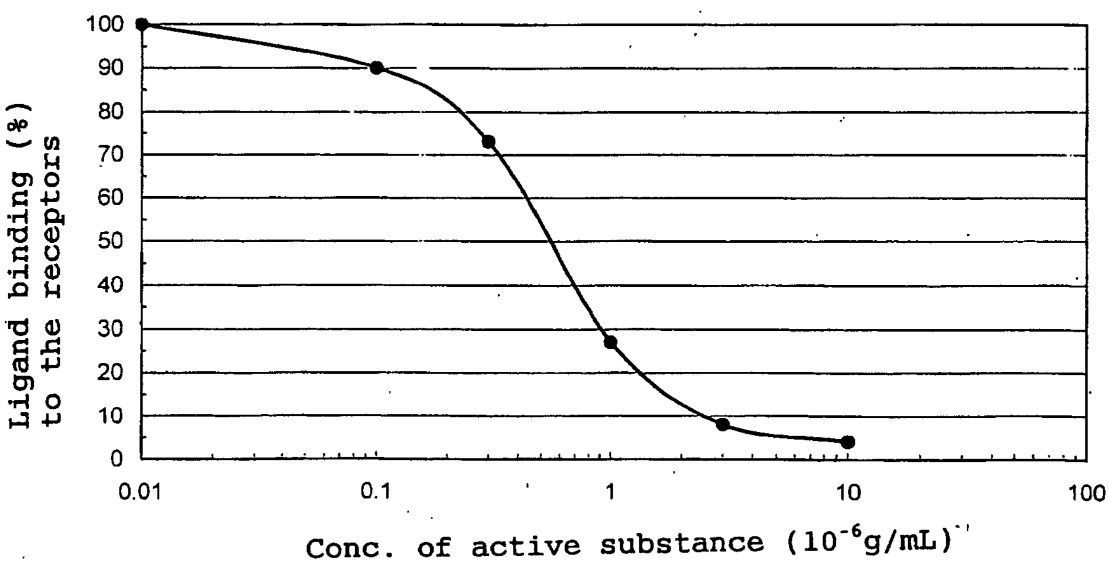
Ligand binding (%)
to the receptors
100
90
80
70
60
50
40
30
20
10
0
0.01
0.1
1
10
100
Conc. of active substance (10-6g/mL)

Fig. 5

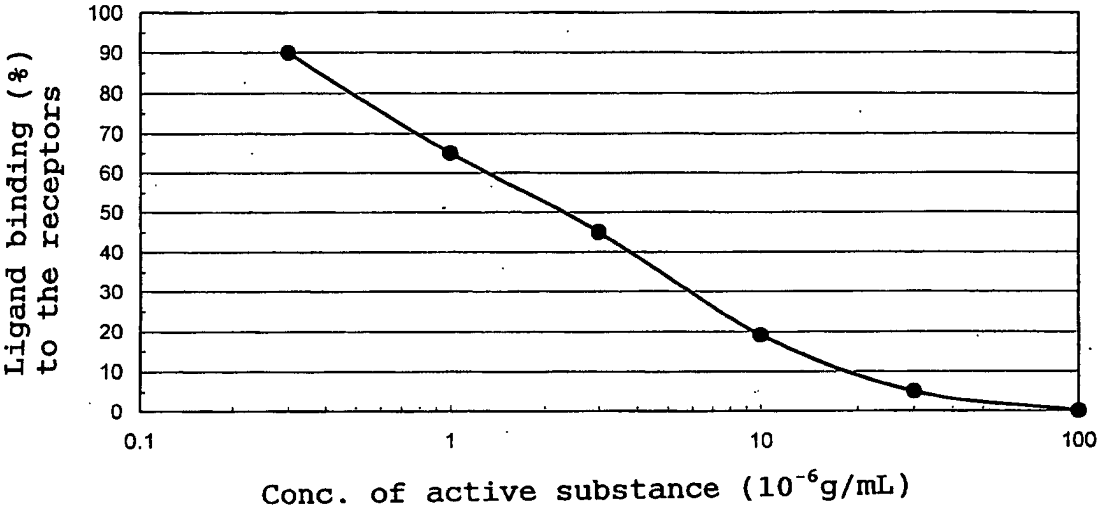
100
90
80
70
60
50
40
30
20
10
0
Ligand binding (%) to the receptors
0.1
1
10
100
Conc. of active substance ($10^{-6}$g/mL)

Fig. 6

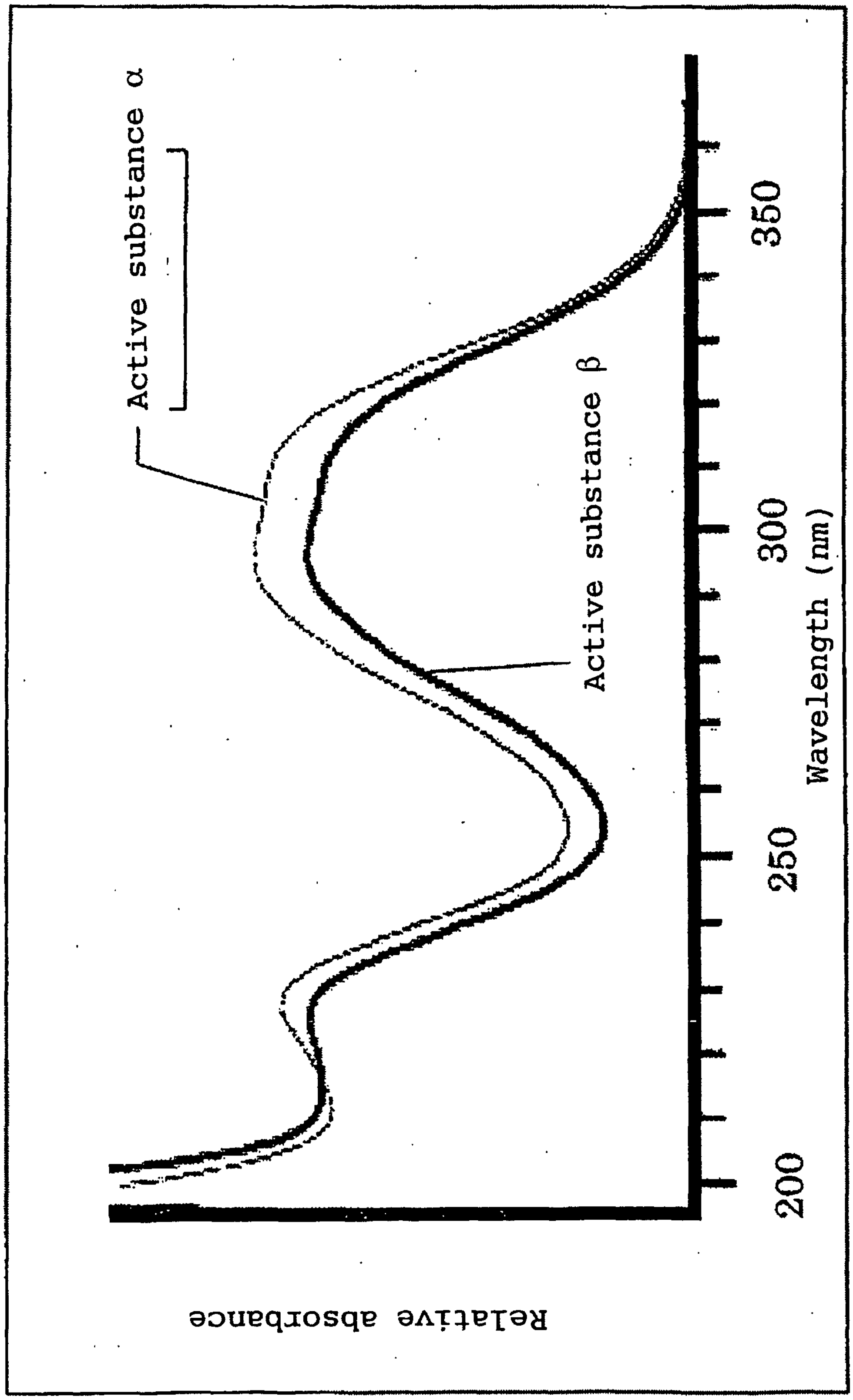
Active substance α
Active substance β
Relative absorbance
Wavelength (nm)
200
250
300
350

Fig. 7

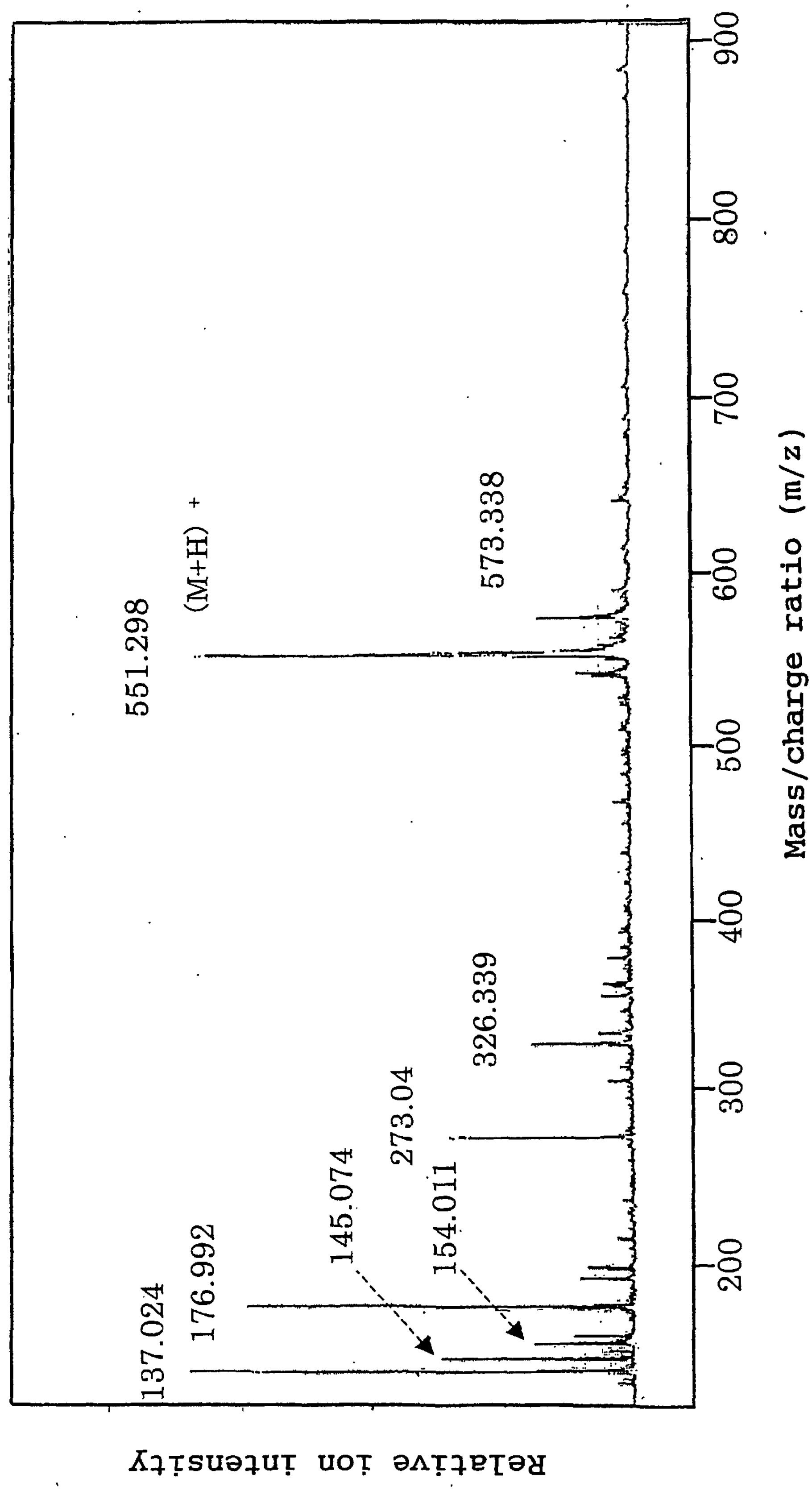
137.024
176.992
145.074
154.011
273.04
326.339
551.298
(M+H) +
573.338
200
300
400
500
600
700
800
900
Mass/charge ratio (m/z)
Relative ion intensity

Fig. 8

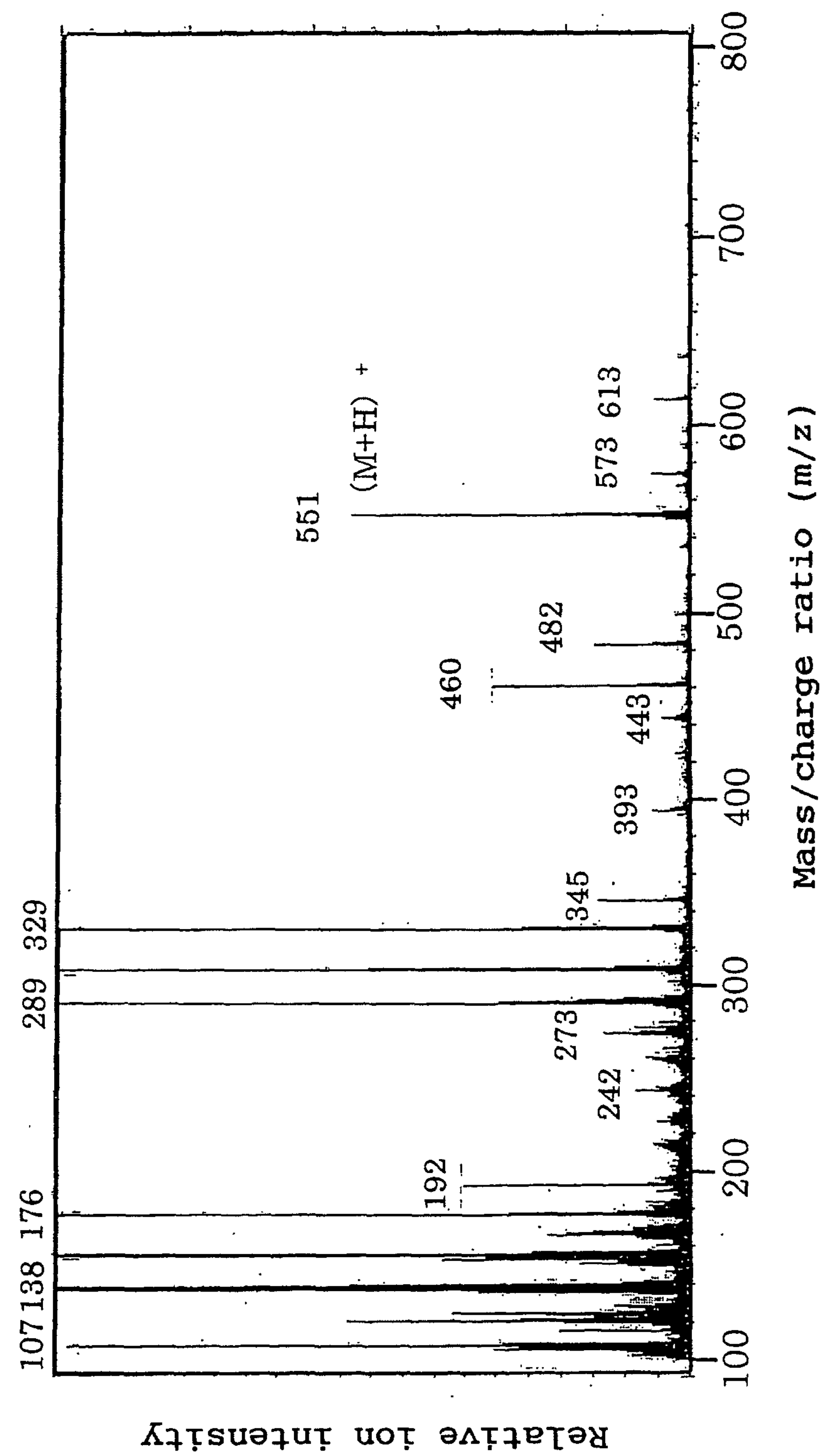
Relative ion intensity
Mass/charge ratio (m/z)
100
200
300
400
500
600
700
800
107
138
176
192
242
273
289
329
345
393
443
460
482
551
(M+H) +
573
613

Fig. 9

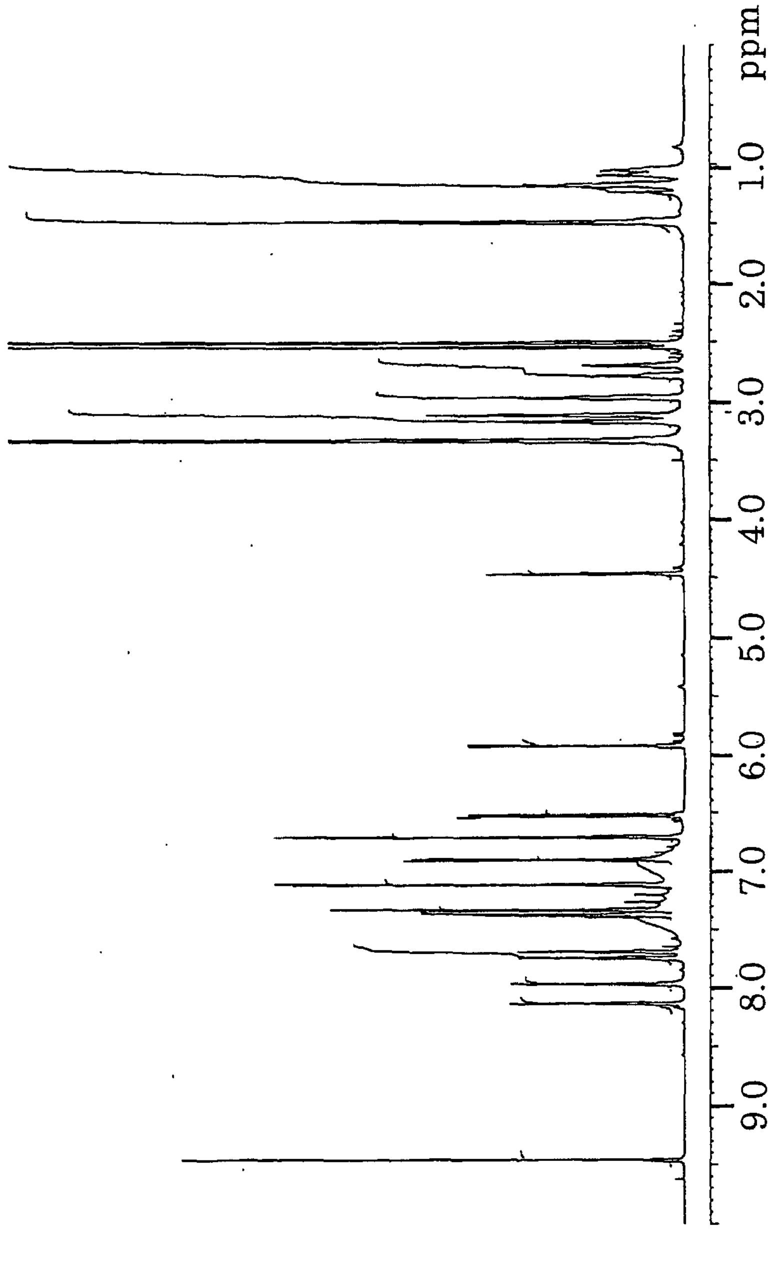

Fig. 10

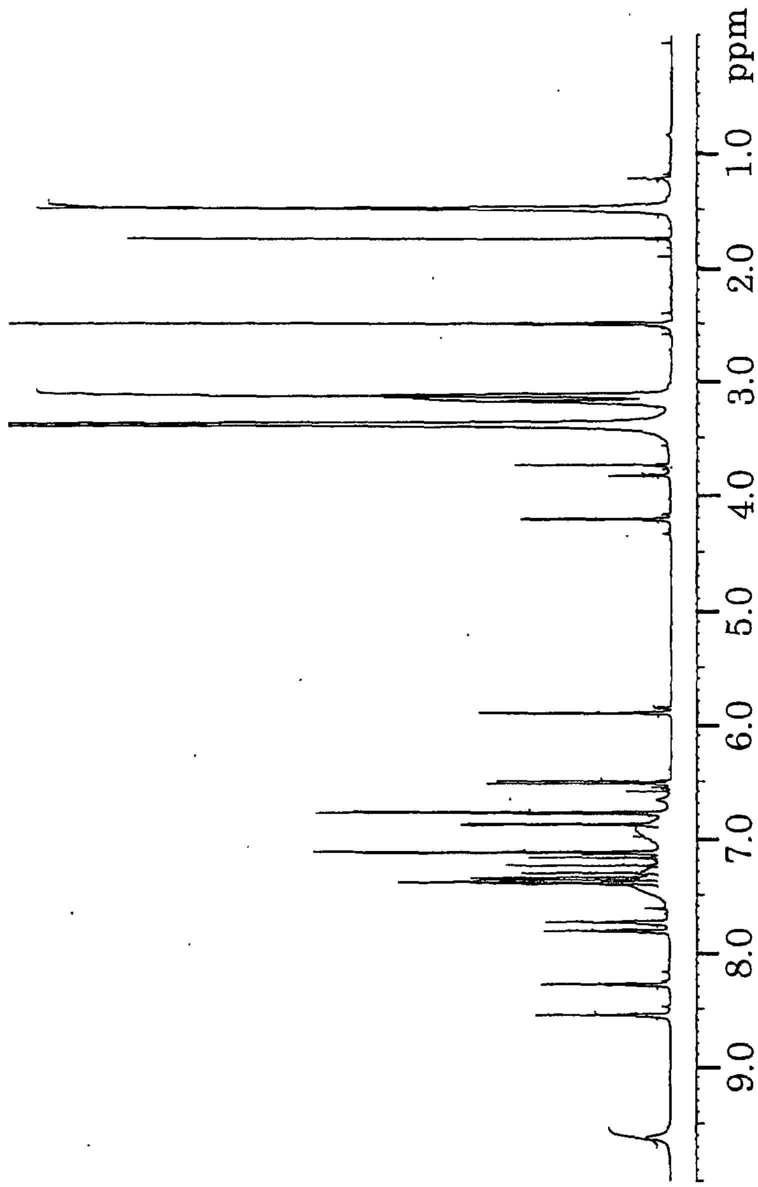

Fig. 11

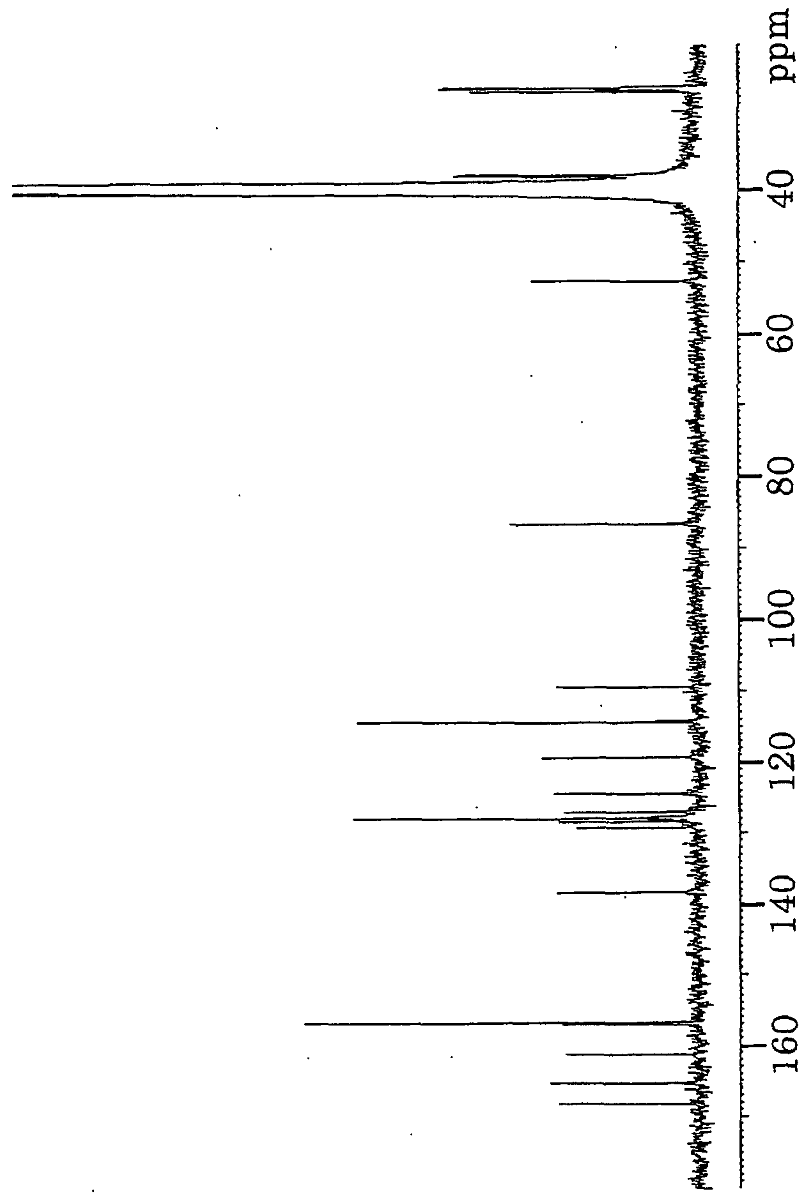

Fig. 12

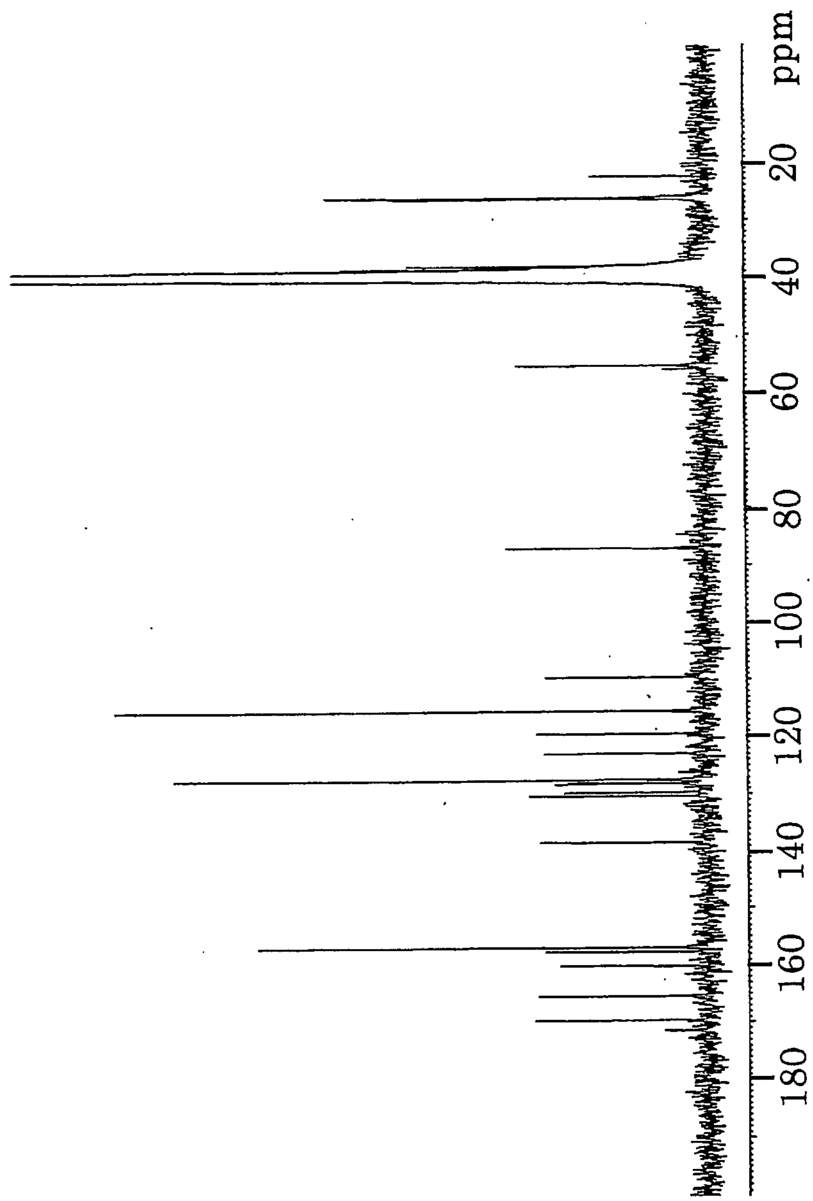

Fig. 13

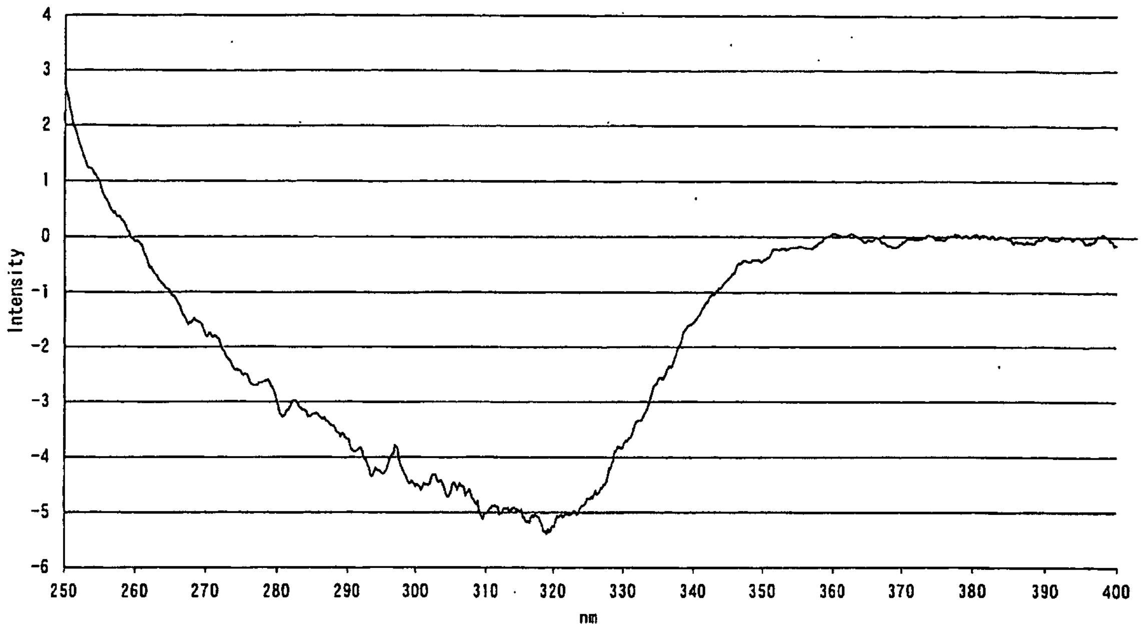
4
3
2
1
0
-1
-2
-3
-4
-5
-6
Intensity
250
260
270
280
290
300
310
320
330
340
350
360
370
380
390
400
nm

Fig. 14

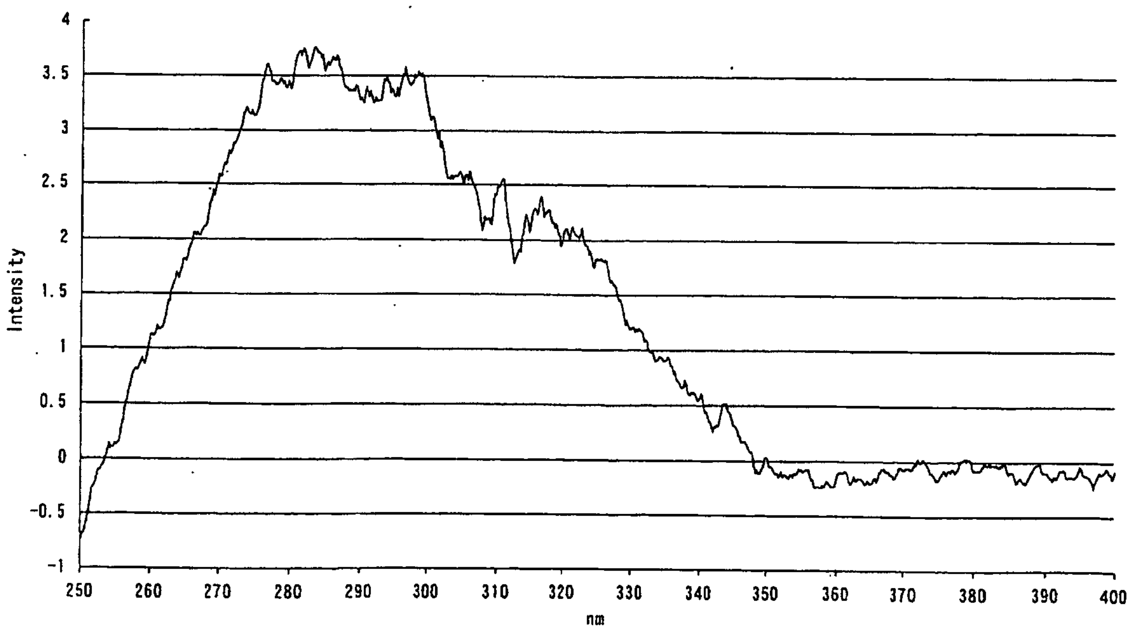
4
3.5
3
2.5
2
1.5
1
0.5
0
-0.5
-1
Intensity
250
260
270
280
290
300
310
320
330
340
350
360
370
380
390
400
nm

Fig. 15

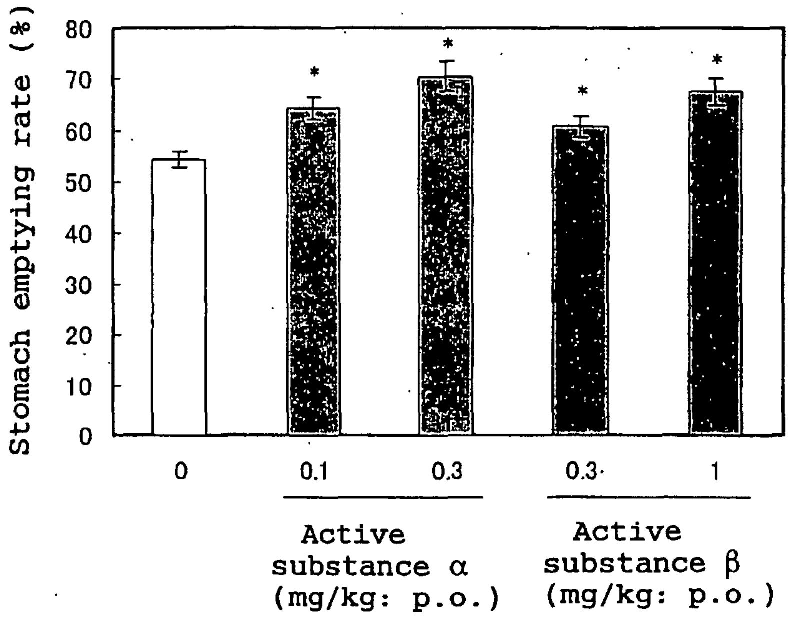
Stomach emptying rate (%)
80
70
60
50
40
30
20
10
0
*
*
*
*
0
0.1
0.3
0.3
1
Active substance α (mg/kg: p.o.)
Active substance β (mg/kg: p.o.)

Fig. 16

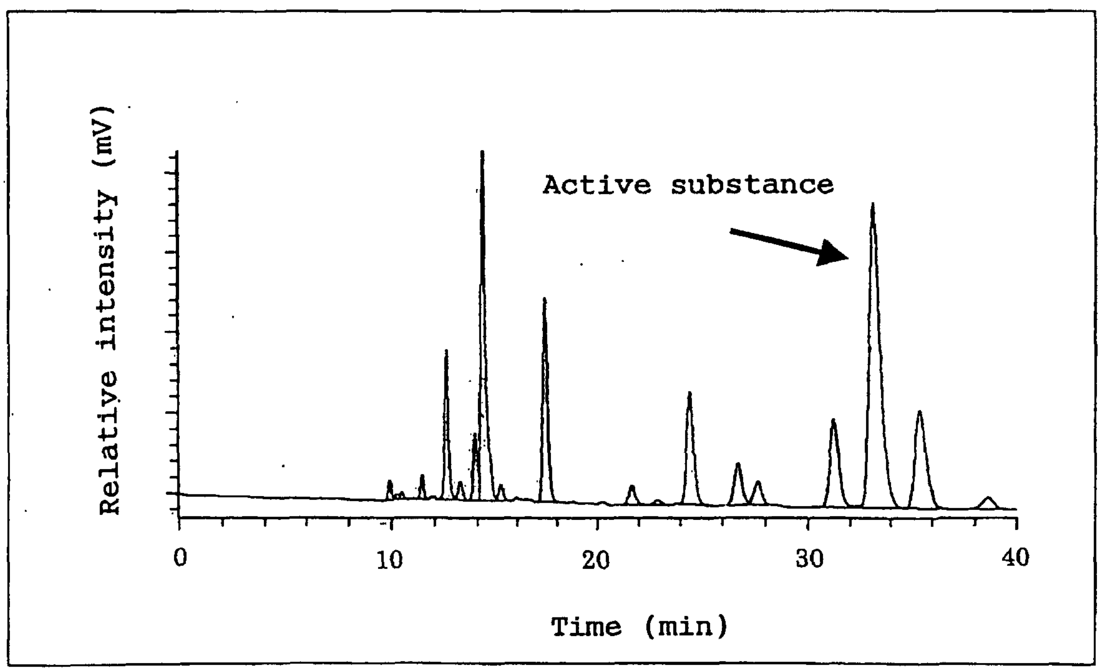
Relative intensity (mV)
Active substance
0
10
20
30
40
Time (min)

Fig. 17

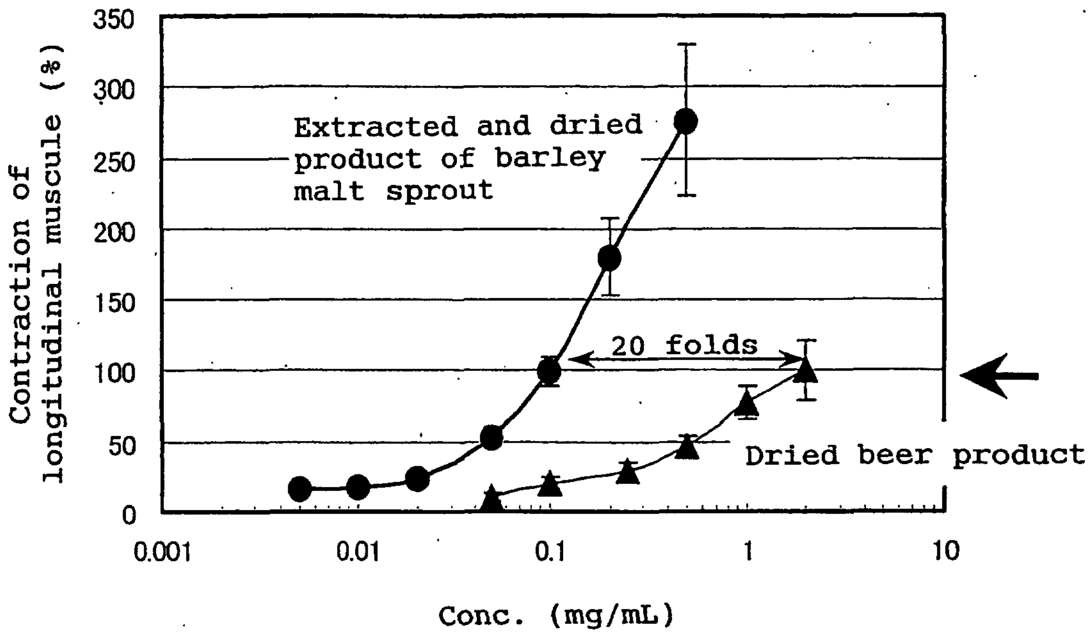
350
300
250
200
150
100
50
0
Contraction of longitudinal muscule (%)
Extracted and dried product of barley malt sprout
20 folds
Dried beer product
0.001
0.01
0.1
1
10
Conc. (mg/mL)

Fig. 18

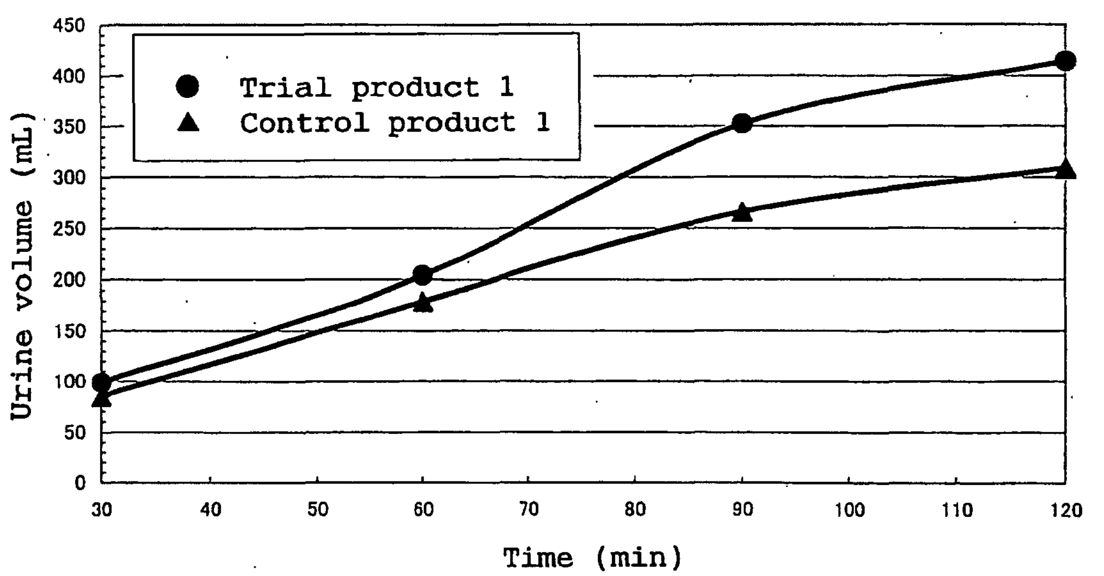
450
400
350
300
250
200
150
100
50
0
Urine volume (mL)
Trial product 1
Control product 1
30
40
50
60
70
80
90
100
110
120
Time (min)

Fig. 19

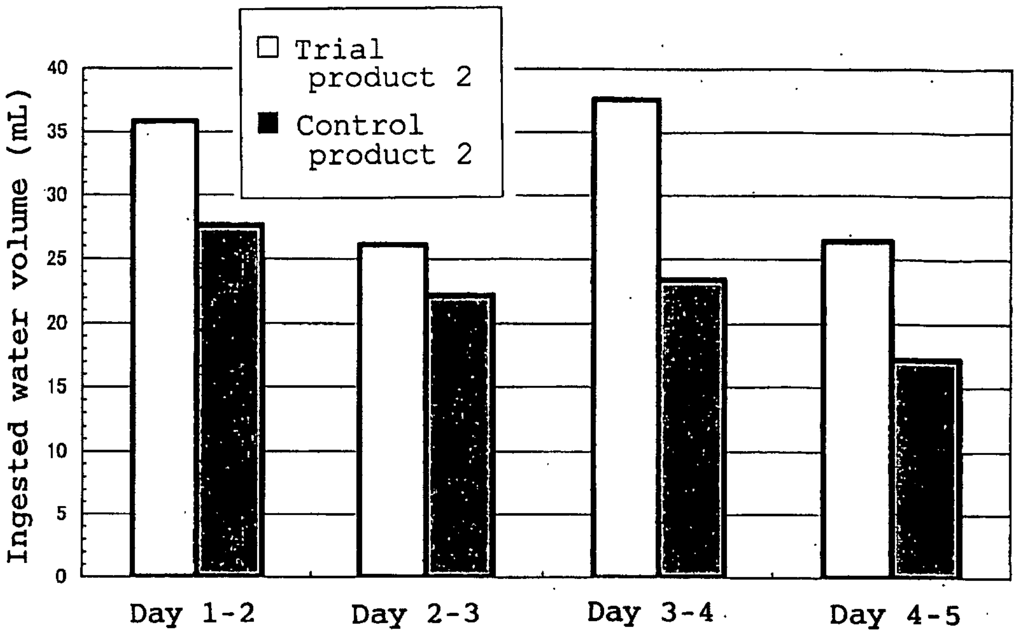
Trial product 2
Control product 2
Ingested water volume (mL)
40
35
30
25
20
15
10
5
0
Day 1-2
Day 2-3
Day 3-4
Day 4-5

Fig. 20

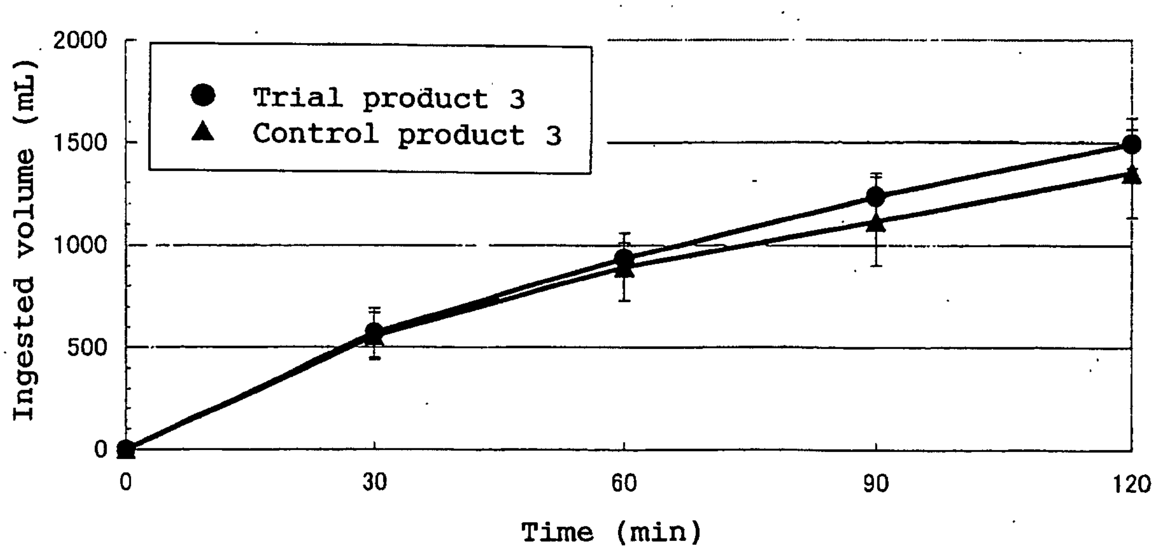
Trial product 3
Control product 3
Ingested volume (mL)
2000
1500
1000
500
0
0
30
60
90
120
Time (min)

**INTERNATIONAL SEARCH REPORT**

International application No.
PCT/JP03/08081

A. CLASSIFICATION OF SUBJECT MATTER
Int.Cl[7] C07D307/84, A61K31/343, A23L1/30, 1/30, 1/28, 2/00, C12C5/00, C12G3/00, A61K35/72, 35/78, A61P1/00, 1/14, 13/02, 43/00

According to International Patent Classification (IPC) or to both national classification and IPC

B. FIELDS SEARCHED

Minimum documentation searched (classification system followed by classification symbols)
Int.Cl[7] C07D307/84, A61K31/343, A23L1/30, 1/30, 1/28, 2/00, C12C5/00, C12G3/00, A61K35/72, 35/78

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | US 3475459 A (STOESSL, A.), 28 October, 1969 (28.10.69), (Family: none) | 1-10<br>11-30 |
| X<br>Y | STOESSL, A. et al., "The antifungal factors in barley. V. Antifungal activity of the hordatines", Canadian Journal of Botany, Vol.48, No.3, 1970, pages 465 to 470 | 1-10<br>11-30 |
| X<br>Y | STOESSL, A., "The antifungal factors in barley. The constitution of hordatines A and B", Tetrahedron Letters, Vol.21, 1966, pages 2287 to 2292 | 1-10<br>11-30 |

[X] Further documents are listed in the continuation of Box C.  [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 August, 2003 (11.08.03) | 02 September, 2003 (02.09.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.
PCT/JP03/08081

C (Continuation). DOCUMENTS CONSIDERED TO BE RELEVANT

| Category* | Citation of document, with indication, where appropriate, of the relevant passages | Relevant to claim No. |
|---|---|---|
| X | FUJII, W. et al., "Beer congener stimulates gastrointestinal motility via the muscarinic acetylecholine receptors.", Alcohol Clin.Exp. Res., Vol.26, No.5, May 2002, pages 677 to 681 | 11-26,29,30 |
| Y | Co-edited by Sainosuke OTSUKA, Mitsuaki MUKAIYAMA, "Fusei Gosei to Kogaku Bunkatsu no Shinpo", Kagaku special extra issue No.97, Kagaku-Dojin Publishing Co., Ltd., 15 October, 1982 (15.10.82), pages 157 to 159 | 27,28 |

Form PCT/ISA/210 (continuation of second sheet) (July 1998)